# FASCICULE DE BREVET EUROPEEN

(11) **EP 1 330 656 B1**
(45) Date de publication et mention de la délivrance du brevet: **16.04.2008**
(21) Numéro de dépôt: 01992896.9
(22) Date de dépôt: 05.11.2001
(51) Int. Cl.: G01N 33/68

(54) **MOYENS DE DETECTION DE LA TRANSFORMATION PATHOLOGIQUE DE LA PROTEINE APP ET LEURS APPLICATIONS**
NACHWEISMITTEL VON PATOLOGISCHEN VERÄNDERUNGEN DES PROTEINS APP UND IHRE VERWENDUNGEN
MEANS FOR DETECTING PATHOLOGICAL TRANSFORMATION OF THE APP PROTEIN AND THEIR USES

(30) Priorité: 03.11.2000 FR 0014143
(43) Date de publication de la demande: 30.07.2003
(73) Titulaire: INSTITUT NATIONAL DE LA SANTE ET DE LA RECHERCHE MEDICALE (INSERM), 75654 Paris Cédex 13 (FR)
(72) Inventeur: DELACOURTE, André, F-59155 FACHES-THUMESNIL (FR); SERGEANT, Nicolas, F-59790 RONCHIN (FR)
(74) Mandataire: Peaucelle, Chantal
(86) Numéro de dépôt international: PCT/FR2001/003410
(87) Numéro de publication internationale: WO 2002/037118

(56) Documents cités:
- EP-A- 0 564 946
- WO-A-92/00521
- WO-A-94/07144
- US-A- 5 234 814
- US-A- 5 441 870
- KOSIK K S: "ALZHEIMER'S DISEASE: A CELL BIOLOGICAL PERSPECTIVE" SCIENCE, AMERICAN ASSOCIATION FOR THE ADVANCEMENT OF SCIENCE,, US, vol. 256, 8 mai 1992 (1992-05-08), pages 780-783, XP002045838 ISSN: 0036-8075
- GANDY S E ET AL: "THE NATURE AND METABOLISM OF POTENTIALLY AMYLOIDOGENIC CARBOXYL-TERMINAL FRAGMENTS OF THE ALZHEIMER BETA/A4-AMYLOID PRECURSOR PROTEIN: SOME TECHNICAL NOTES" NEUROBIOLOGY OF AGING, TARRYTOWN, NY, US, vol. 13, no. 5, 1992, pages 601-603, XP000614997 ISSN: 0197-4580
- SUZUKI T ET AL: "PHOSPHORYLATION OF ALZHEIMER AMYLOID PRECURSOR PROTEIN BY PROTEIN KINASE C" NEUROSCIENCE, vol. 48, no. 4, 1992, pages 755-761, XP001053372 ISSN: 0306-4522

## Description

L'invention se rapporte à des moyens de détection de la transformation pathologique de la protéine APP (pour Amyloid Precursor Protein) dans des maladies neurodégénératives telle que la maladie d'Alzheimer.

Elle vise plus particulièrement une méthode de détection d'une telle transformation, les kits pour sa mise en oeuvre, ainsi que ses applications thérapeutiques et diagnostiques.

La maladie d'Alzheimer (MA) est une maladie neurodégénérative qui conduit à la perte des fonctions intellectuelles et donc à l'installation progressive et irréversible d'une démence. L'avenir de cette maladie s'annonce redoutable avec le vieillissement de la population car le facteur de risque majeur est l'âge.

99% des formes observées sont non-familiales. Pour les formes familiales autosomiques dominantes, les mutations pathologiques sont observées sur le gène APP du chromosome 21, et sur les gènes PS1 et PS2 (présénilines 1 et 2) qui se trouvent sur les chromosomes 14 et 1.

Deux processus dégénératifs caractérisent la maladie d'Alzheimer. L'amyloidogénèse, qui résulte d'un dysfonctionnement de la protéine APP, et la dégénérescence neurofibrillaire (DNF), qui correspond à l'accumulation de protéine tau dans les cellules nerveuses.

Le dysfonctionnement de la protéine APP est à l'origine même de la pathologie Alzheimérienne, mais la cause précise de la dégénérescence et de la mort neuronale ne sont pas encore connues.

Deux hypothèses ont été émises. Selon la première hypothèse, qui est la plus répandue, le produit de clivage de l'APP, le peptide Aβ, de 39 à 43 résidus d'acides aminés, est neurotoxique et responsable de la réaction en chaîne de DNF .

Selon la seconde hypothèse, la dégénérescence résulterait d'une perte ou d'un gain de fonction de l'APP, qui se traduirait parallèlement et secondairement par une accumulation du peptide Aβ sous forme de plaques amyloïdes.

La modélisation de l'amyloïdogénèse est possible grâce aux souris transgéniques avec les gènes mutés APP ou APP+PS1. Ces souris développent progressivement des plaques amyloïdes, mais on n'observe pas de DNF. Ces souris sont déjà utilisées par l'industrie pharmaceutique pour tester des molécules capables d'inhiber la formation du peptide Aβ. La stratégie principale consiste à bloquer les enzymes responsables de la production du peptide Aβ. Les enzymes de ce type connues à ce jour sont les secrétases β et γ (1).

Des molécules se fixant sur les dépôts amyloides sont également testées. Il s'agit de casseurs de structures β (β sheet breakers) ou anti- amyloides (2).

La vaccination contre le peptide amyloïde est également en cours d'évaluation (3).

A l'heure actuelle, aucune modification significative et confirmée de la protéine APP dans le tissu humain affecté par la maladie d'Alzheimer n'a été découverte, autre que l'augmentation de la production du peptide Aβ 1-42, l'augmentation du rapport Aβ 1-42/1-40 et son agrégation sous forme de dépôts insolubles et de plaques amyloïdes.

Une modification de la sécrétion de la partie N- terminale de l'APP, nommée sAPP, a également été signalée dans des essais in vitro testant la mutation 715 sur l'APP (4).

US5,234,814 décrit une augmentation de la quantité des fragments APP-Cter dans le fluide cérébrospinale des patients avec la maladie d'Alzheimer. WO9407144 décrit une augmentation de la quantité des fragments APP-Cter présentant des modifications post-traductionnelles, en particulier la phosphorylation, dans l'épithélium des patients avec la maladie d'Alzheimer, et suggère une détection basée sur l'électrophorèse bidimensionnelle.

Les recherches menées par les inventeurs les ont amené à observer des modifications significatives de la protéine APP qui ne sont pas liées à la détection des peptides Aβ, et qui sont spécifiquement détectables dans le tissu humain Alzheimérisé et dans les modèles expérimentaux

Ces modifications sont mises en évidence dans le tissu humain (système nerveux central, liquides et tissus périphériques) affecté par la maladie d'Alzheimer, ainsi que dans d'autres pathologies neurodégénératives avec dysfonctionnement de l'APP.

Les modifications significatives sont à la fois qualitatives et quantitatives et elles sont détectées en amont de la production du peptide Aβ.

Des anomalies similaires peuvent être retrouvées en grande quantité chez les souris transgéniques avec les mutations pathologiques sur l'APP ou APP mutée + préséniline 1 mutée.

L'invention vise donc l'utilisation de ces modifications observées dans le tissu cérébral humain comme marqueurs dans une méthode de détection de la transformation pathologique de APP, notamment dans un processus neurodégénératif.

Elle vise l'application de ces marqueurs notamment pour le diagnostic et le suivi d'un processus neurodégénératif, la mise au point de modèles animaux et le criblage de médicaments efficaces contre les pathologies de l'APP.

L'invention vise également une méthode d'évaluation desdites pathologies comprenant la définition d'un indice établi par rapport à un référentiel.

La méthode pour détecter, dans un échantillon à analyser, des modifications pathologiques de la protéine APP est caractérisée en ce qu'on utilise des marqueurs constitués par des fragments cataboliques et/ou métaboliques de la partie carboxy terminale de l'APP, appelés ci-après fragments APP-Cter, spécifiquement modifiés dans les situations pathologiques neurodégénératives où l'APP participe à l'étiologie.

Les études réalisées par les inventeurs ont montré que, de manière inattendue, les modifications concernant de tels fragments étaient directement liées à l'installation et à la progression du processus dégénératif dans les régions cérébrales, notamment dans le cas de la maladie d'Alzheimer.

On observera que les fragments en question sont des produits cataboliques et/ou métaboliques de l'APP qui ont conservé la structure primaire de l'extrémité C-terminale de l'APP et seront donc reconnaissables par un anticorps dirigé contre cette partie C-terminale de l'APP.

Le terme "fragment" tel qu'utilisé dans la description et les revendications englobe donc les variants de charge et de point isoélectrique différents suite à des modifications post-traductionnelles, comme par exemple la phosphorylation et/ou la méthylation et/ou l'acétylation et/ou la glycosylation, dans la mesure où ces variants sont reconnus par un anticorps anti-APP-Cter comme décrit dans les exemples.

Il s'agit notamment de fragments tels qu'identifiés par analyse biochimique d'un échantillon à analyser, par électrophorèse 1-D (électrophorèse monodimentionnelle) ou 2-D (électrophorèse bidimentionnelle) couplée à une immunoempreinte et révélation par un jeu d'anticorps polyclonaux et/ou monoclonaux anti-APP-Cter, et en complément par un jeu d'anticorps contre les modifications post-traductionnelles de la transformation pathologique de l'APP-Cter.

L'électrophorèse peut être mono et/ou bidimensionnelle.

De tels fragments sont spécifiquement modifiés dans les tissus pathologiques. On note la diminution, voire la disparition de certains de ces fragments avec la progression du processus neurodégénératif. Parallèlement, on note une modification de la charge électrique de certains de ces fragments et une modification de leur solubilité.

L'invention vise spécialement l'application de cette méthode au diagnostic du processus d'Alzheimérisation et, à ce titre, vise l'utilisation comme marqueurs de dysfonctionnements de l'APP, de 5 fragments majeurs qui sont modifiés au cours de l'Alzheimérisation, dont les poids moléculaires déterminés par électrophorèse monodimensionnelle (1-D) sont de 14, 5 ; 13,5 ; 12 ; 10,5 et 9,5 kDa. Par commodité, ces fragments sont désignés par les lettres A, B, C, D, E. Il est à noter qu'une bande de 9 kDa (F) est également présente et une bande de 6,5 kDa, en quantités moindres et variables.

Les variants qui disparaissent correspondent à la bande électrophorétique 1-D de 14,5 kDa, nommée A, dédoublée en deux composantes de 15 kDa et 14,5 kDa en 2-D, avec des variants isoélectriques de points isoélectriques de 5,13 (représenté par 15/5,13) et 15/5,37 pour la composante de 15 kDa et de 14,5/5,64 et 14,5/5,95 pour l'autre composante. Il y a également disparition de fragments correspondant à la bande électrophorétique B de 13,5 kDa qui est dédoublée en deux composantes de 13,5 et 12,7kDa, avec les variants isoélectriques 13,5/4,94 et 12,7/4,8; 12,7/5,29.

Des fragments d'intérêt sont également modifiés en phase précoce de la maladie d'Alzheimer. Ils correspondent aux variants électrophorétiques 15/4,55; 14/5,2; 14/5,37; 12,7/5,45; 12,7/5,64; 10,5/5,91; 12,7/6,05. Ces variants sont particulièrement informatifs dans la mesure où ce sont des marqueurs précoces.

Les fragments qui apparaissent au cours de l'Alzheimérisation correspondent à la bande électrophorétique 1-D de 10,5 kDa, dédoublée en deux composantes de 11 kDa et 10,5 kDa en 2-D, avec des variants isoélectriques 11/4,94; 10,5/5,35 et 10,5/7,5 et le fragment de 9,5 kDa et de pI 5,77.

Parallèlement, des modifications de solubilité des marqueurs APP-Cter sont également observées au cours de l'évolution du processus pathologique de neurodégénérescence. Les fragments de 14,5 et 13,5 voient leur solubilité diminuer et les fragments de 10,5 et 9,5 voient leur solubilité augmenter au cours de l'Alzheimérisation du tissu cérébral.

De tels fragments sont modifiés dans les tissus ou cellules dits alzheimérisés. Ils peuvent être modifiés d'une manière similaire dans des modèles expérimentaux, tels les animaux transgéniques portant un gène ou une combinaison de gènes mutés ou non mutés, impliqués dans la pathologie d'Alzheimer.

On notera avec intérêt que ces différences de catabolisme et de métabolisme de la protéine APP, tels qu'observés avec les marqueurs APP-Cter, sont observées dans les modèles cellulaires neuronaux (par exemple les cellules de neuroblastome comme la souche SKNSH SY5Y, la souche NT2, la souche Kelly), les souches de glioblastome (CCF, U118, T98) mais également dans des cellules non neuronales, comme les souches CHO, COS, HT-29, et Hela.

On notera également que chaque lignée cellulaire possède naturellement un patron d'expression de ces fragments qui lui est propre (variation de la quantité globale des fragments de masse moléculaire 13,5 ; 12 ; 10,5 ; 9,5 ; 9 ; 8,5 et 6,5 kDa exprimés par rapport aux protéines totales du modèle cellulaire; variations du rapport entre chaque fragment.

Pour ces modèles, et d'une manière spécifique à chaque modèle, l'expression des fragments A-F est également modifiée en fonction de l'état de différenciation des souches cellulaires.

Ces variations sont également observées dans les modèles animaux, notamment dans les souris transgéniques avec les différentes constructions possibles (mutations APP et/ou mutations PS1 ou PS2 et/ou absence d'expression de PS1) et/ou gène tau muté ou non muté (14,5 ; 13,5 ; 12 ; 10,5 ; 9,5 ; 9 ; 8,5 et 6,5 kDa).

Les échantillons à analyser utilisés dans ladite méthode comprennent donc aussi bien des tissus ou des souches cellulaires neuronales, que des tissus ou des souches cellulaires non neuronales, par exemple les liquides biologiques comme le liquide céphalo-rachidien, le sang et tout ou partie de ses éléments figurés, ce qui facilite un diagnostic précoce, l'évaluation de facteurs de risque chez un patient ou le suivi thérapeutique.

Ces tissus sont avantageusement homogénéisés ou fractionnés, par exemple par centrifugation, en vue de leur mise en oeuvre dans la méthode de détection.

La méthode ci-dessus s'applique également au diagnostic différentiel de pathologies neurodégénératives voisines de la maladie d'Alzheimer, comme la démence à corps de Lewy ou les angiopathies amyloïdes.

La méthode de détection définie ci-dessus permet de définir un indice de transformation de la protéine APP en protéine pathologique.

L'invention vise en particulier une méthode d'évaluation d'un processus pathologique neurodégénératif dans un échantillon à analyser, par analyse immuno-chimique, comme décrit dans les exemples, caractérisé en ce qu'on affecte un indice aux fragments identifiés dans l'échantillon étudié, par rapport à un référentiel où l'indice 0 (zéro) correspond à la détection dans le tissu sain et l'indice 100 (cent) dans le tissu pathologique, qu'il soit humain, de souris transgéniques avec des mutations des gènes incriminés dans la pathologie Alzheimer, ou de souches cellulaires natives ou transfectés avec lesdits gènes.

Un tel indice présente un avantage en diagnostic et en thérapeutique. Il permet de refléter rapidement l'état pathologique. La valeur de l'indice permet en effet de déterminer s'il existe un risque de développement de la maladie d'Alzheimer. L'obtention d'un indice élevé peut aider à conforter un diagnostic clinique.

En outre, dans le cadre d'un traitement thérapeutique basé sur le catabolisme et le métabolisme de l'APP ou sa correction, les modifications d'un tel indice permettront de suivre l'effet du traitement.

Il constitué une référence permettant de comparer l'effet de différentes molécules contre la maladie d'Alzheimer ou autres pathologies neurodégénératives, une modification de l'indice démontrant l'efficacité des molécules testées et les potentialités de leur intérêt thérapeutique.

L'utilisation d'un tel indice présente également un intérêt pour disséquer les évènements étiologiques précoces responsables de la maladie, et aboutir à la découverte de nouvelles molécules efficaces, ainsi qu'au développement de nouvelles stratégies thérapeutiques.

On notera également que cet indice peut être utilisé pour ralentir ou même pour induire une dégénérescence pour établir de nouveaux modèles expérimentaux. Cet indice peut aussi fournir des informations permettant de provoquer une dégénérescence ciblée dans le cadre d'actions thérapeutiques nécessitant de provoquer la disparition d'une population cellulaire précise.

La méthode selon l'invention peut avantageusement utiliser en complément un jeu d'anticorps polyclonaux et/ou monoclonaux afin de révéler des modifications post-traductionnelles de la transformation pathologique des fragments APP-Cter, en particulier une modification de type phosphorylation, et/ou méthylation et/ou acétylation et/ou glycosylation.

Préférentiellement, le jeu d'anticorps polyclonaux et/ou monoclonaux utilisé permet de révèler les phosphorylations et la variation d'expression des fragments de 14,5 ; 12 et 10,5 kDa.

La méthode de détection selon l'invention est avantageusement utilisable pour établir et valider des modèles expérimentaux, cellules ou modèles animaux de la maladie d'Alzheimer. Les modèles pertinents reproduiront les modifications des fragments APP-Cter tels qu'observés dans le tissu humain, comme décrit dans les exemples.

Elle permet également d'aider à la mise en place d'essais thérapeutiques efficaces en améliorant la sélection des patients.

Une autre application de grand intérêt concerne le criblage pharmacologique pour la sélection de médicaments efficaces contre les pathologies neurodégénératives de type Alzheimer. Ainsi, les molécules sélectionnées sont caractérisées en ce qu'elles sont capables de modifier le profil Alzheimer évoqué ci-dessus, en conduisant à une restoration des propriétés des marqueurs APP-Cter, ou à une restoration de leurs propriétés physico-chimiques (solubilité, charge électrique).

L'invention permet ainsi de réaliser un criblage pharmacologique rapide et pertinent de molécules d'intérêt.

L'invention vise également un kit de diagnostic de la transformation pathologique de APP, caractérisé en ce qu'il comprend un jeu d'anticorps polyclonaux et/ou monoclonaux dirigé contre la région carboxy-terminale de l'APP. L'utilisation d'un jeu d'anticorps dirigé contre les modifications post-traductionnelles liées à la transformation pathologique située sur des fragments APP-Cter, comme par exemple des sites de phosphorylation, méthylation, acétylation ou de glycosylation sur ces fragments, pourra avantageusement apporter des informations complémentaires sur la mise en évidence et la quantification de la transformation pathologique des fragments APP-Cter.

L'hétérogénéité des fragments APP-Cter démontrée par les électrophorèses 2D est expliquée en partie par les modifications de phosphorylation, comme le démontre un anticorps polyclonal phospho-dépendant dirigé contre la thréonine 668 phosphorylée (nomenclature avec l'APP 695), située dans une région commune aux fragments APP-Cter.

Elles résultent également de glycosylation, comme le montre les drogues anti-glycosylation qui modifient le profil des fragments APP-Cter. La méthylation et l'acétylation sont deux modifications post-traductionnelles qui sont également impliquées dans la transformation pathologique des fragments APP-Cter. En effet, elles sont localisées essentiellement sur des résidus lysine, ce qui provoque une acidification et donc un changement de point isoélectrique.

Elles sont également la résultante d'un contrôle par des gènes impliqués dans la maladie d'Alzheimer. C'est ainsi que la surexpression de la protéine Tau est capable d'influencer le rapport des bandes de 13,5 ; 9,5 et 6,5 kDa.

Ces anticorps sont utilisables dans un dosage immuno-chimique par exemple en ELISA, Western blot, dot blot. Les kits selon l'invention renfermeront donc avantageusement les éléments nécessaires pour la réalisation de tels dosages.

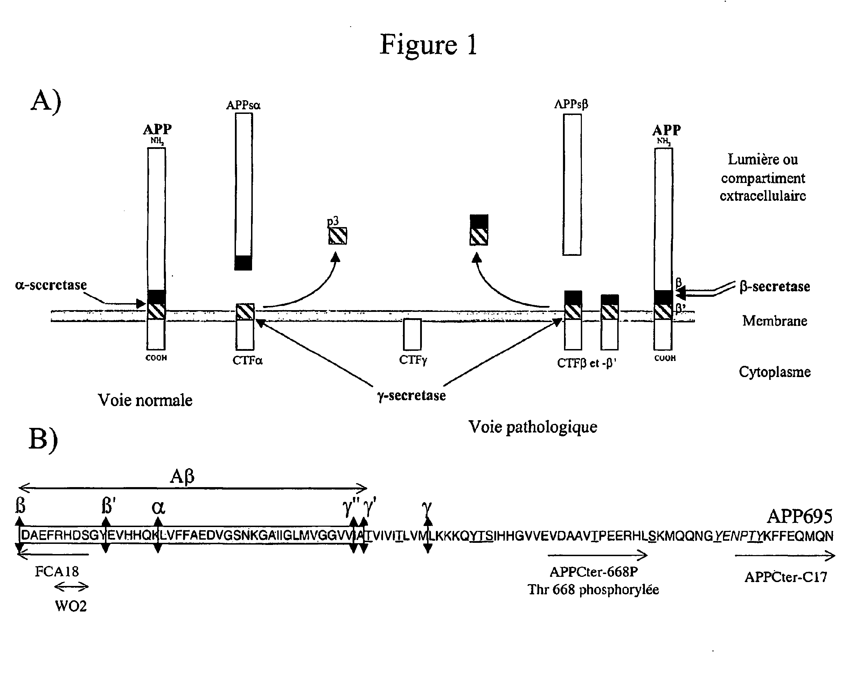

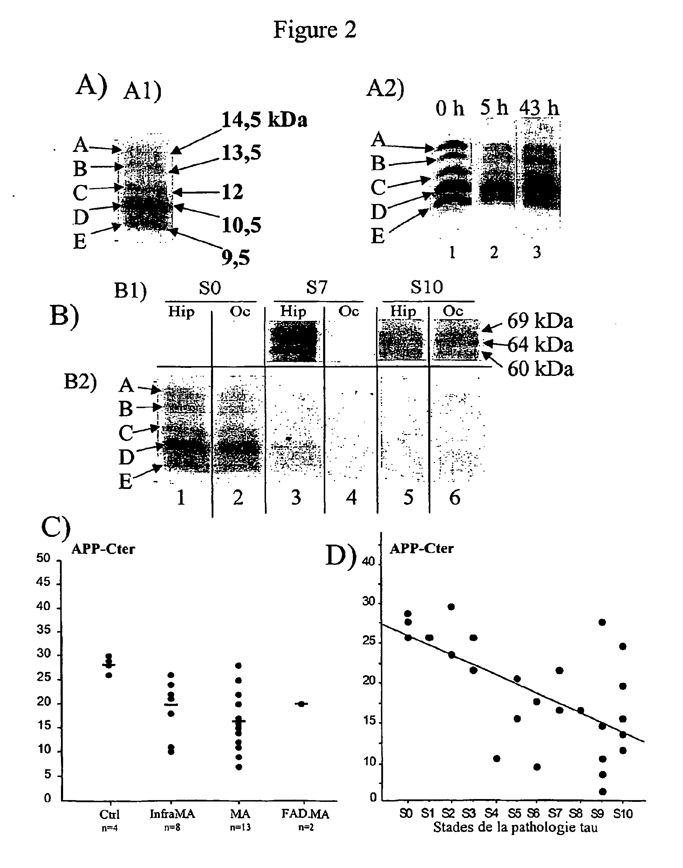

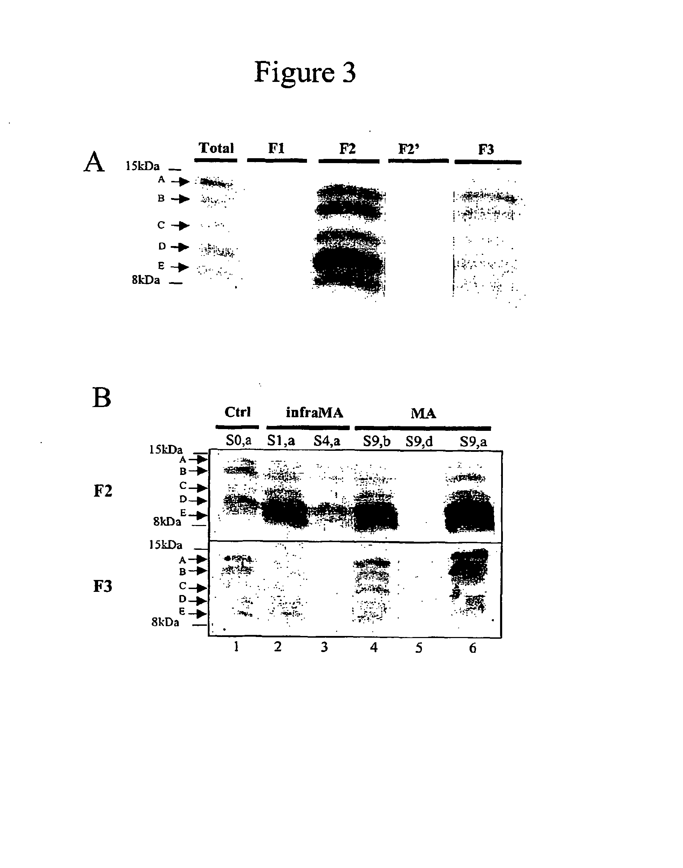

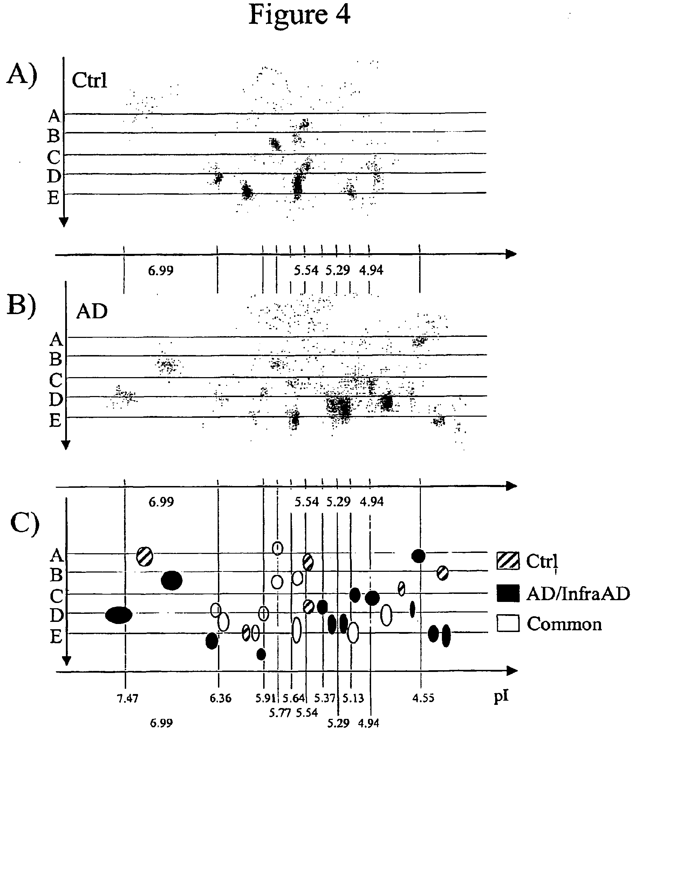

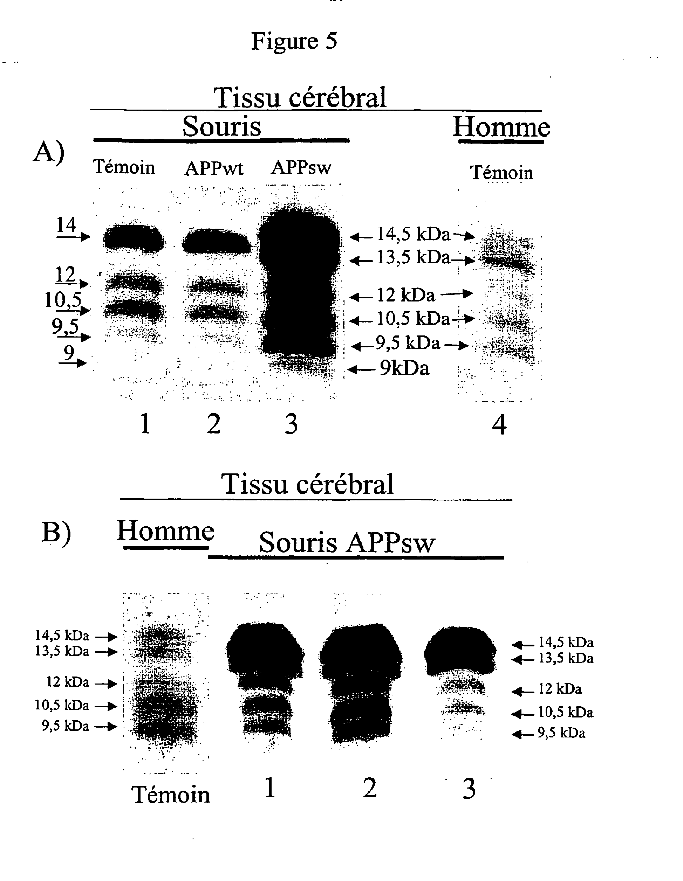

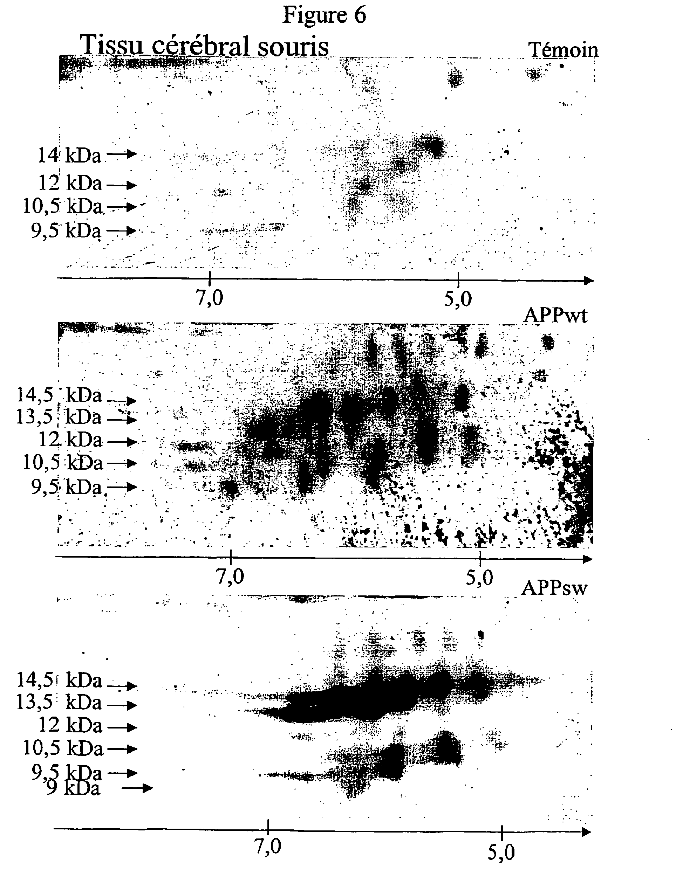

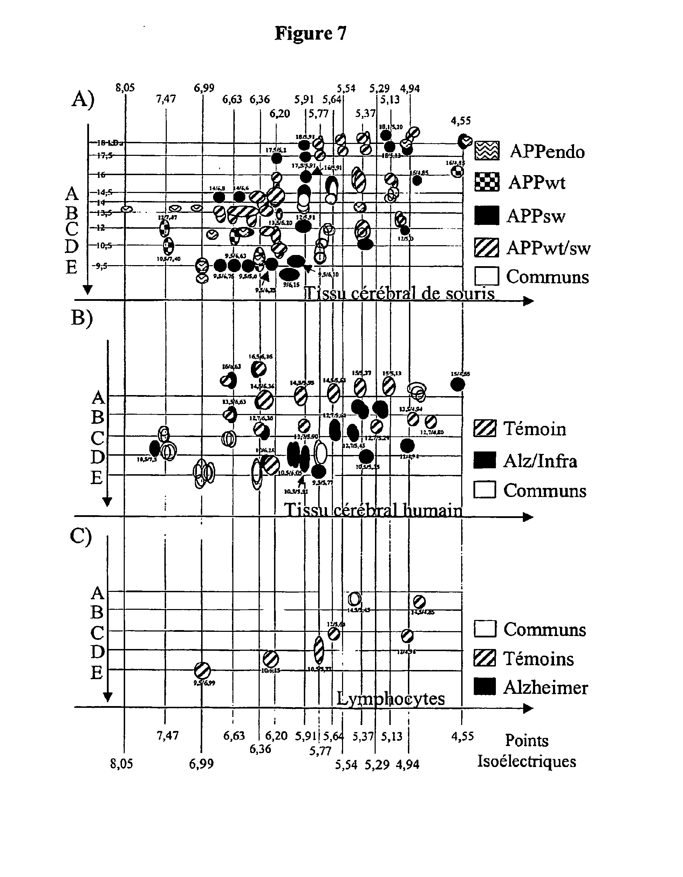

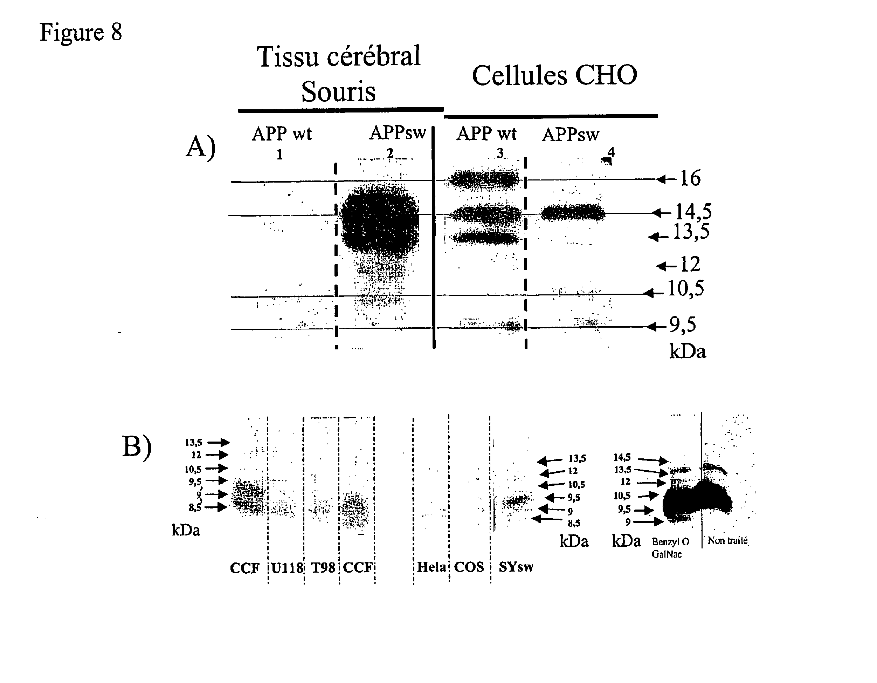

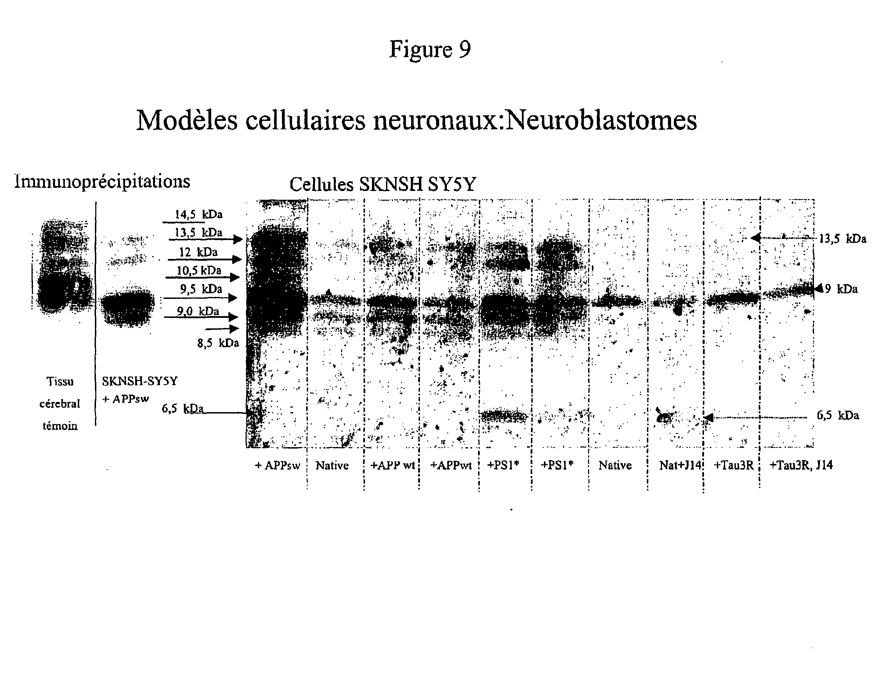

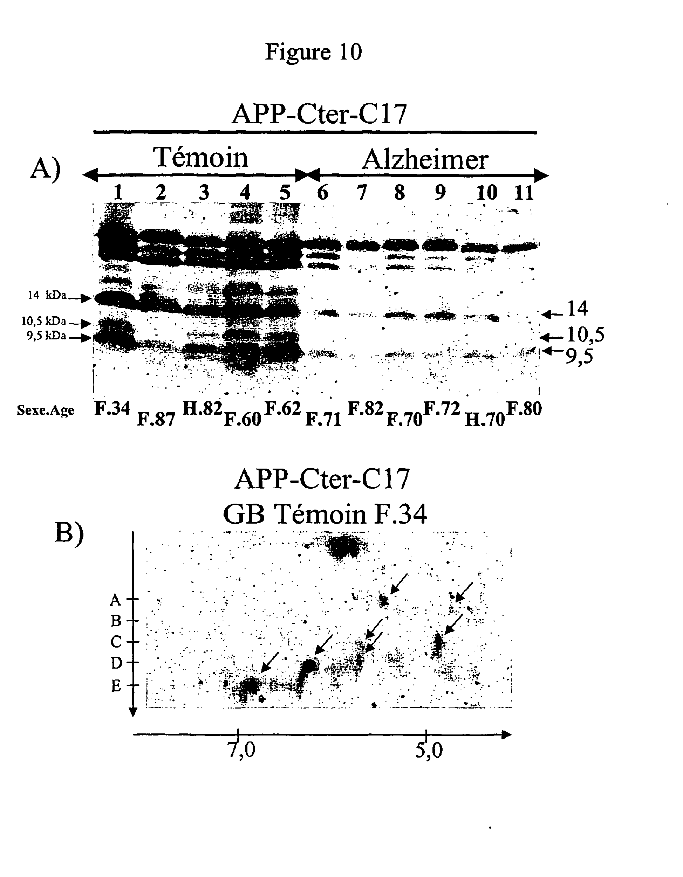

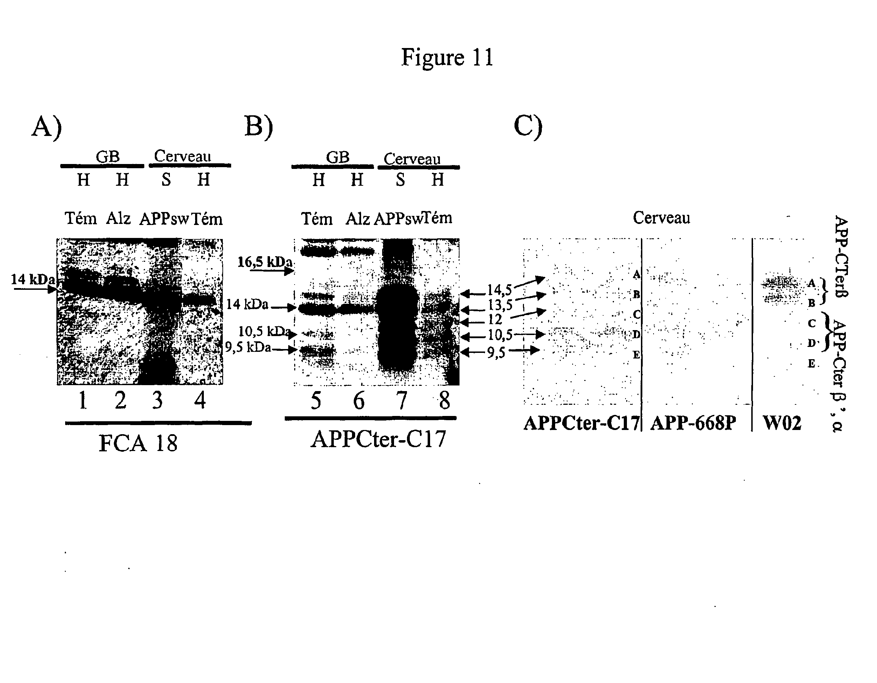

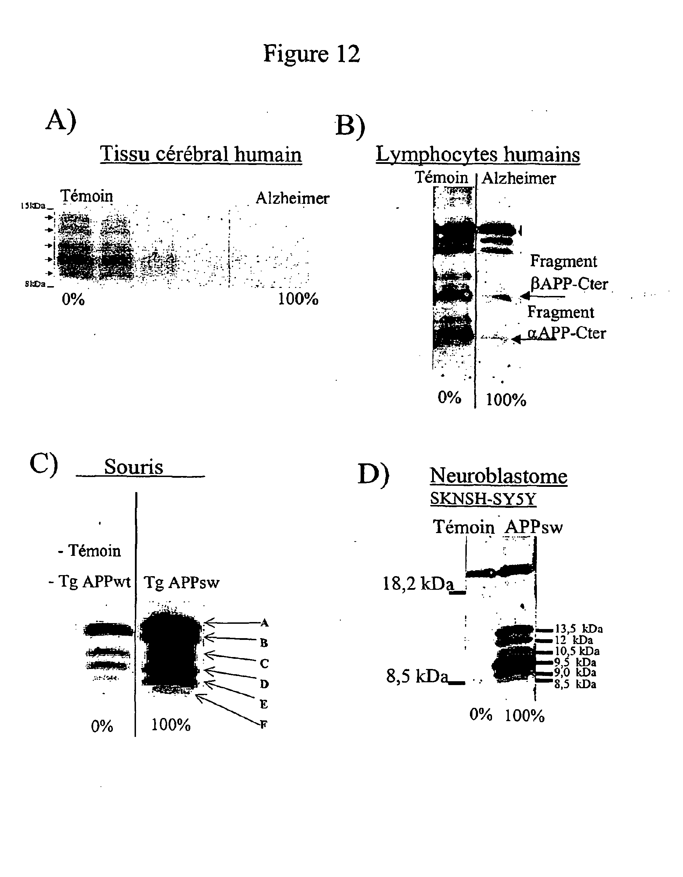

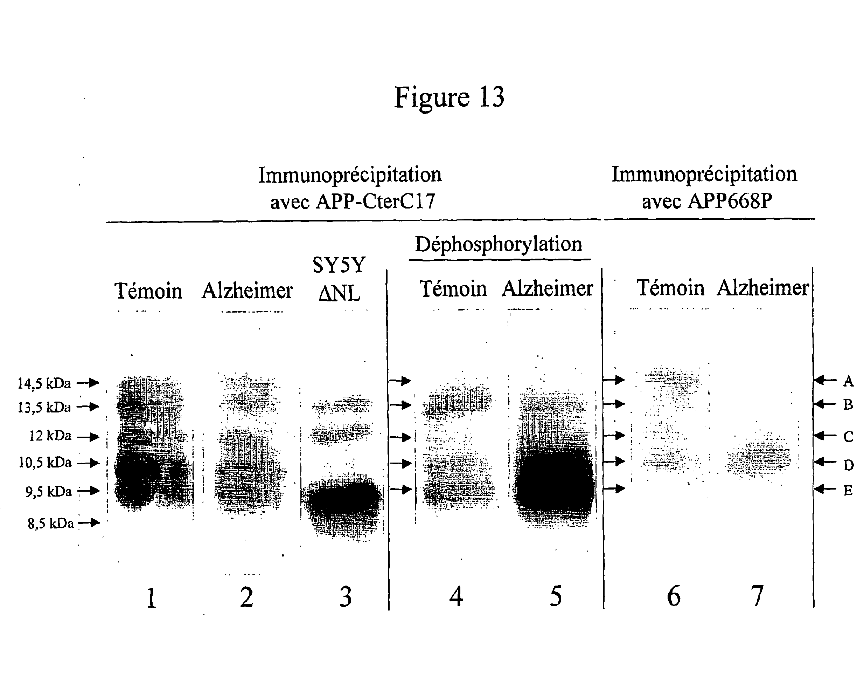

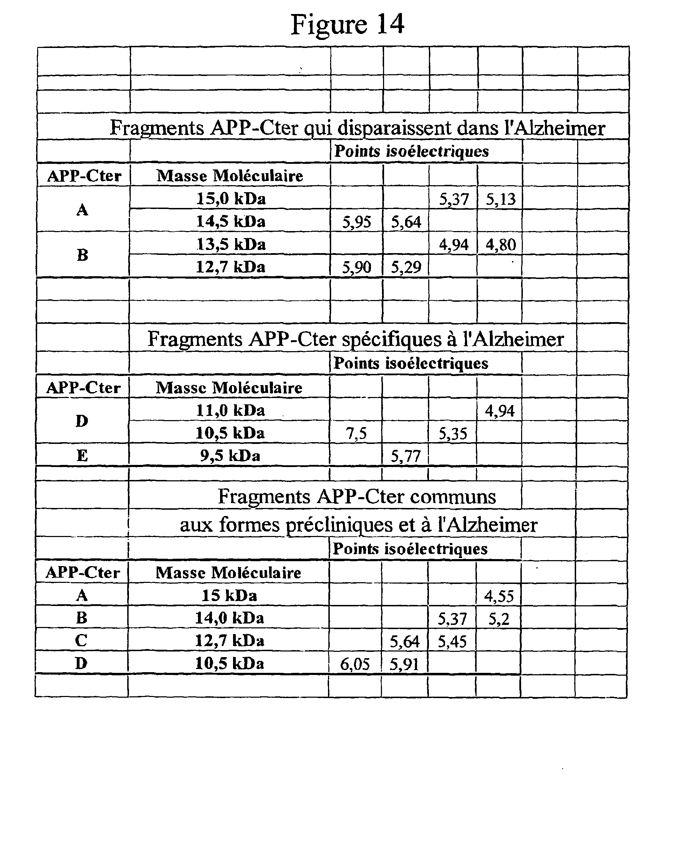

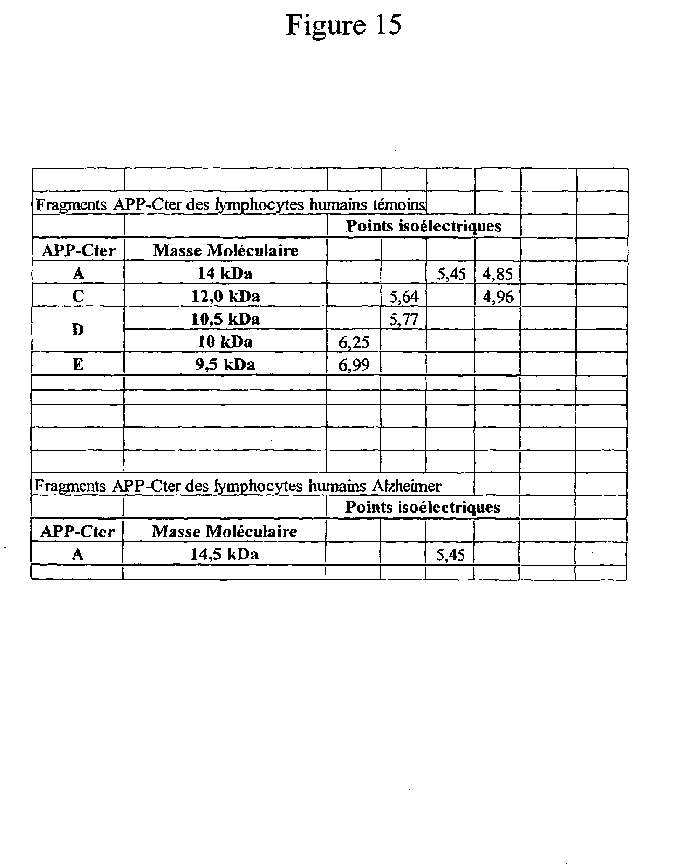

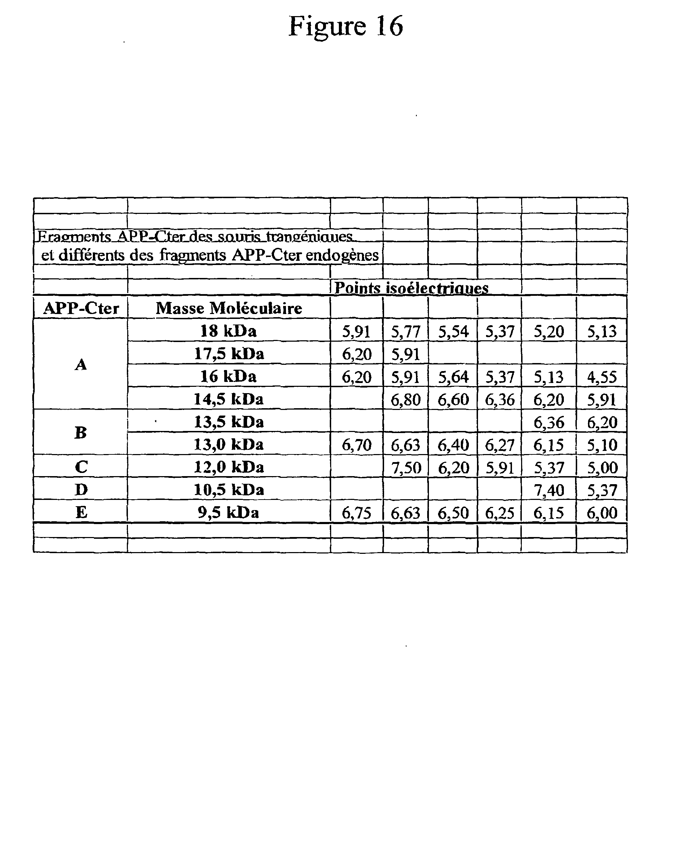

D'autres caractéristiques et avantages de l'invention apparaîtront dans les exemples qui suivent, avec référence aux figures 1 à 16, qui représentent, respectivement,
- le schéma théorique de coupure des fragments APP-Cter et localisation des épitopes des anticorps utilisés(figure 1)
- les photos d'empreintes Western de
   - tissu cérébral humain (1-D) (figures 2A à 2B), et dosage des fragments APP-Cter (fig 2C et 2D).
   - fractionnement du tissu cérébral humain (figure 3A et 3B)
   - tissu cérébral humain (2-D) (figures 4A à 4C),
   - tissu cérébral de souris transgéniques (1-D) (figures 5A et 5B),
   - tissus cérébral de souris (2-D) (figure 6),
- une synthèse des profils 2-D de fragments APP-Cter dans les souris, le tissu cérébral humain et les lymphocytes humains (respectivement figures 7A, 7B, 7C),
- les photos d'empreintes Western de
   - tissu cérébral de souris WT et SW, des souches cellulaires non-neuronales avec APP wt et sw (figure 8),
   - comparaison du tissu cérébral humain avec les souches cellulaires de type neuronal transfectées ou non avec APP wt, sw, PSl muté, Tau 3R ou 4R(figure 9),
   - lymphocytes humains : analyses 1-D et 2-D (figures 10A et 10B),
- la caractérisation des fragments APP-Cter avec des anticorps anti-Aβ-N-terminal, APP-Cter et un anticorps APP-Cter phospho-dépendant (figure 11)
- l'indice de transformation pathologique (figures 12 A à D)
- l'influence de la phosphorylation sur la transformation pathologique des APP-Cter (figure 13)
- des tableaux récapitulatifs des variants selon l'invention (figures 14 à 16).

### MATERIELS ET METHODES

### Anticorps

### Production de l'anticorps polyclonal APP-Cter-C17

Un anticorps polyclonal dirigé contre la partie carboxy-terminale de l'APP a été produit selon le protocole d'immunisation décrit par Vaitukaitis et al, (5). Des lapins New Zealand ont été immunisés avec un peptide comprenant les 17 derniers acides aminés de la séquence de l'APP humaine, correspondant à son extrémité carboxy-terminale. Le peptide a été couplé par covalence à l'ovalbumine. 200 µg d'immunogène ont été injectés toutes les deux semaines. Avant l'injection, la solution d'immunogène a été complétée avec 1 volume d'adjuvant complet de Freund lors du 1er rappel et lors des rappels suivants, avec de l'adjuvant incomplet de Freund à la place de l'adjuvant complet. Le sang a été prélevé une semaine après la 4ème injection et une semaine après chaque injection suivante. Le sérum pur a été obtenu après coagulation du sang et centrifugation 10 min à 2000g. On a ajouté au sérum pur 1 volume de glycérol et on a conservé le mélange à -20°C.

### Purification de l'anticorps APP-Cter-C17

1 mg de peptide utilisé pour l'immunisation a été couplé à une matrice NHS-Fast Flow Sepharose® (Amersham-Pharmacia Biotech) suivant les instructions du fabricant. On a mis en contact la matrice couplée avec 200 µl de sérum pur dilué dans un volume de tampon de liaison 2X (50 mM Tris pH 8, 0 ; 300 mM NaCI et 0, 1 % (v/v) de Tween-20) et on a agité doucement jusqu'au lendemain à 4°C. On a fait passer la totalité des solutions sur une colonne PD-10. On a lavé la matrice avec 10 volumes de tampon de liaison. L'anticorps purifié a été élué avec 2 volumes de tampon acétate à pH 3,5. La concentration en protéine a été déterminée à l'aide du kit de quantification de protéine BCA de Pierce.

### Production de l'anticorps polyclonal APP-668P

Un anticorps dirigé contre un peptide synthétique correspondant à la séquence Val-Asp-Ala-Ala-Ala-Val-Thr(phosphorylé)-Pro-Glu-Glu-Arg-His-Leu a été produit, en suivant le même protocole que celui utilisé pour l'APP-Cter-C17. Cet anticorps reconnaît l'immunogéne, mais il ne reconnaît pas un peptide ayant la même séquence, mais non phosphorylé.

### Echantillons

### Echantillons de tissu cérébral humain et murin

Le tissu cérébral humain provient de patients qui ont été suivis prospectivement, et décrits dans (6). Les échantillons de tissu cérébral, obtenus après autopsie ou biopsie et conservés à - 80°C, ont été disséqués à l'aide d'un atlas anatomique, puis homogénéisés avec un potter en Téflon® dans 10 volumes de tampon de lyse 1-D 1X (50 mM Tris-HCl pH 6,8 ; 4mM EDTA ; 5% (p/v) de SDS, 10 % (v/v) de glycérol, 2 % (v/v) de β-mercaptoéthanol et 0,05% Bleu de Bromophénol) pour une analyse 1-D. Les échantillons ont été portés à 100°C pendant 10 minutes puis maintenus à -80°C jusqu'à leur utilisation. Pour une analyse 2-D, le tissu est homogénéisé dans un tampon Tris-HCl 10 mM pH 6,8, puis complémenté avec un volume de tampon 2-D 2X (7M urée ; 2M thiourée; 0,4% Pharmalytes® 3-10 (p/v); 8% (v/v) Triton X-100; 10 mM de dithiothréitol; 0,1% Bleu de Bromophénol) et stocké à -80°C jusqu'à utilisation. Pour les échantillons de tissu cérébral de rongeur, le cerveau a été prélevé rapidement après la mort de l'animal et traité de la même manière que les échantillons humains.

### Lignées murines utilisées.

On a préparé des échantillons de tissu cérébral murin en suivant le même protocole que pour les échantillons de tissu cérébral humain. On a utilisé des échantillons de tissu cérébral de souris non transgéniques et de souris transgéniques avec le gène APP humain de type sauvage (APPwt) ou le gène humain APP muté aux codons 670 et 671 (APPsw). Cette mutation de l'APP est liée à une forme familiale de la maladie d'Alzheimer, appelée mutation suédoise (7). D'autres échantillons ont été également utilisés, comprenant des souris non transgéniques, et différentes lignées de souris transgéniques avec le gène APPwt et APPsw. On a également utilisé un échantillon de tissu cérébral murin similaire à la souris transgénique développée par Hsiao K. et al. (8) .

### Echantillons de tissu cérébral après fractionnement protéique

Les échantillons de tissu cérébral ont été homogénéisés selon un ratio 1/10 (p/v) dans du tampon Tris-HCl 10 mM pH 6,8 puis centrifugés à 100 000 g pendant 1 heure à 4°C. Le surnageant (F1) a été conservé et le culot ré-homogénéisé dans le même tampon complémenté avec 0,5 % (v/v) de Triton X-100. Après centrifugation, la fraction Triton X100 soluble, nommée fraction F2, est stockée. Une étape supplémentaire d'extraction et de centrifugation dans le même tampon a été effectuée dans les mêmes conditions, ce qui donne la fraction F2'. Le culot est ensuite directement repris dans le tampon de lyse 1-D (voir ci-dessous) et correspond à la fraction F3.
Selon la technique biochimique utilisée, on a ajouté aux surnageants, au moment de l'utilisation, les tampons correspondants: 1 volume de tampon de lyse 1-D 2X (100 mM Tris-HCl pH 6,8 ; 8mM EDTA ; 10% (p/v) de SDS ; 20 % (v/v) de glycérol ; 4 % (v/v) de β-mercaptoéthanol et 0,1% Bleu de Bromophénol) pour une étude par électrophorèse monodimensionnelle (1-D) ou 1 volume de tampon de lyse 2-D (7M urée ; 2M thiourée ; 0,4% Pharmalytes® 3-10 (p/v) ; 4% (v/v) Triton X-100 ; 10 mM de dithiothréitol ; 0,1% Bleu de Bromophénol) pour une étude par électrophorèse bidimensionnelle (2-D).

### Immunoprécipitation

La fraction F2 est utilisée pour immunoprécipiter les fragments APP-Cter. 100 µl de la fraction F2 sont dilués dans 300 µl de tampon Tris 10 mM pH 7,4 contenant 1% de NP-40 (Tampon d'immunoprécipitation). 10 µl d'anticorps APP-CterC17 ou APP668P sont ajoutés et l'ensemble et incubés sous agitation durant la nuit à 4°C. 40µl de protéine A fixée sur des billes d'agarose (Pierce) sont ajoutés à la solution et l'ensemble est incubé sous agitation à 4°C pendant une heure. Les billes d'agarose sont lavées trois fois dans le tampon d'immunoprécipitation puis les fragments APP-Cter sont décrochés par un traitement avec 50µl de tampon SDS et récupérés dans le surnageant de centrifugation. Les fragments APP-Cter sont ensuite analysés par immuno-empreinte après électrophorèse en gel Tris-Tricine.

### Echantillons de cultures cellulaires CHO (hamster) ou SKNSH SY5Y (humain)

Les cellules sont transfectées de manière stable avec le gène APP humain de type sauvage ou le gène APP humain portant la mutation suédoise. Le culot de cellules a été repris dans un tampon Tris-HCl 10mM pH 6,8 puis passé aux ultrasons. Avant utilisation, l'échantillon est complété avec 1 volume de tampon de lyse correspondant à l'expérience (1-D ou 2-D) 2X. Dans le cas d'une analyse 1-D, l'échantillon est porté 10 min à 100°C.

### Echantillons de globules blancs humains

Préparation des globules blancs humains: 10 millilitres de sang sont collectés dans des tubes EDTA (tétra-acétate d'éthylène diamine). On procède à une centrifugation à 4500 rpm (rotation par minute) pendant 15 minutes. Le plasma est éliminé. Le tube est rempli au deux tiers d'une solution de lyse des globules rouges (solution de lyse : NH₄CO₃ 0,91 mM et NH₄Cl 0,132 mM). On agite délicatement le tube et il est placé au bain-marie réfrigéré à 5°C. On procède à une centrifugation à 4000 rpm pendant 15 minutes et le surnageant est éliminé. Le culot de globules blancs est rincé deux fois avec la solution de lyse suivant la même méthodologie. Le culot est ensuite égoutté et, il est soit utilisé dans les 24 heures, soit congelé à -20°C pour son transport ou son utilisation ultérieure.
Le culot de globules blancs est repris dans un tampon Tris-HCl 10mM pH 6,8 puis passé aux ultrasons. Avant utilisation, l'échantillon est complété avec 1 volume de tampon de lyse 2X correspondant à l'expérience (1-D ou 2-D). Dans le cas d'une analyse 1-D, l'échantillon est porté 10 min à 100°C.

### Techniques Electrophorétiques

### Electrophorèse 1-D

Les expériences ont été réalisés à l'aide du système d'électrophorèse Protean IIXi Cell (Biorad) selon les instructions du fabricant.

Les électrophorèses 1-D ont été réalisées selon les protocoles décrits par Laemmli (9) pour la fabrication du gel et dans les conditions de migrations décrits par Schägger et Von Jagow (10). Le gel de concentration contient 4% d'acrylamide et le gel de séparation utilisé contient 16,5% d'acrylamide. La migration se fait en tampon Tris-Tricine. Le programme de migration utilisé est le suivant : 1h à 30V constant puis 16h à 45mA .
On a chargé une quantité équivalente de protéine dans chaque puit (environ 100 µg/puit).

### Electrophorèse 2-D

### 1^{ère} dimension

La première dimension ou focalisation est réalisée à l'aide des bandelettes de gels pré-coulés nommées IPG-Strip® couvrant un gradient de pH 3-10, selon les instructions du constructeur. Le matériel utilisé pour l'isoélectrofocalisation est le système IEF Protean Cell (Biorad).

Une quantité de 250 µg de protéines est complétée avec du tampon de réhydratation 1X (7M urée, 2M thiourée, 4% triton X100; 0,5% CHAPS; 0,2 % (p/v) de Pharmalytes® pH 3-10; 10mM DTT; 0,01% orange G) pour avoir 400µl à déposer. Les 400µl d'échantillons sont déposés sur les bandelettes placées dans l'appareil. On laisse réhydrater passivement 1 h puis activement 10h à 50V/bandelette puis on lance le programme selon les instructions du fabricant. Après la focalisation, les bandelettes sont utilisées ou conservées à -80°C.

### 2^{ème} dimension

Avant utilisation, les bandelettes sont équilibrées pendant 30 minutes dans un tampon pour 1-D (50 mM Tris pH 6,8 ; 10 % (v/v) de glycérol ; 2 % (v/v) de β-mercaptoéthanol ; 2 % (p/v) de SDS ; 0.05% (p/v) bleu de bromophénol) puis déposées sur le haut d'un gel de séparation pour 1-D de 16,5% acrylamide. On recouvre la bandelette avec une solution d'agarose 1% (p/v). La suite est identique à une simple électrophorèse 1-D.

### Transfert sur membrane et Immunoempreinte

### Transfert

Le transfert a été effectué en utilisant le système de transfert semi-sec Pharmacia LKB multiphor® en suivant les instructions du fabricant (Amersham-Pharmacia Biotech). Les protéines ont été transférées à 0,8 mA/cm² sur une membrane de nitrocellulose Hybond® ECL (Pharmacia-Amersham).

### Immunoempreinte

La membrane est incubée 60 min. dans du tampon (Tris 15 mM pH 8,0 ; NaCl 150 mM ; 0,5 % (v/v) de Tween®-20) contenant 5% (p/v) de lait écrémé puis lavée avec le même tampon exempt de lait contenant 0,1% de Tween®-20 au lieu de 0,5%.
La membrane est incubée, 2h à température ambiante ou 1 nuit à 4°C, avec l'anticorps APP-Cter-C17 dilué au 1/2000ème (v/v) final dans un tampon d'incubation (15 mM Tris pH 8,0, 150 mM NaCl, 0,1 % (v/v) de Tween-20 et 3 % (p/v) de lait écrémé).
La membrane est lavée 3 fois pendant 10 minutes dans le tampon d'incubation exempt de lait.
La membrane est ensuite incubée, 1h à température ambiante, avec une immunoglobuline de chèvre anti-lapin couplée à la peroxydase de raifort, à une dilution finale de 1/4000ème (v/v) dans du tampon d'incubation exempt de lait.
La membrane est lavée 3 fois pendant 10 minutes dans du tampon d'incubation exempt de lait et les polypeptides immunoréactifs sont révélés à l'aide du kit de chimiluminescence ECL® (Pharmacia-Amersham Biotech) selon les instructions du fabricant.

### RESULTATS

L'analyse théorique des coupures cataboliques et/ou métaboliques de l'APP est présentée dans la figure 1A et la localisation des épitopes des anticorps utilisés sur la figure 1B.

### Analyse 1-D du tissu cérébral humain

Des échantillons de tissu cérébral humain, préparés comme décrit dans la méthodologie pour une analyse 1-D, ont été chargés et analysés. La séparation électrophorétique a eu lieu en condition Tris-Tricine comme décrit. Des marqueurs de poids moléculaires calibrés (Biorad) ont été déposés en parallèle afin de déterminer la masse moléculaire des fragments grâce au logiciel ImageMaster® 1-D Elite (Amersham-Pharmacia Biotech).
Un transfert des protéines séparées par électrophorèse et une immunoempreinte avec l'anticorps APP-Cter-C17 ont été réalisés comme décrit. Les résultats sont représentés sur la figure 2, Al.

### Analyse du tissu normal.

Dans le tissu cérébral humain témoin, cinq bandes nommées A, B, C, D et E de masses moléculaires apparentes A 14,5 k-Da; B 13,5 kDa; C 12 kDa; D 10,5 kDa ; E 9,5 kDa sont révélées par l'anticorps polyclonal APP-Cter-C17. Elles sont détectées aussi bien dans la biopsie de tissu cérébral humain que dans le tissu cérébral post-mortem d'un sujet témoin (figure 2, A2; piste 1: biopsie; pistes 2 et 3: autopsie). Le délai post-mortem est indiqué en heure pour chacun des échantillons. On peut noter que le profil des fragments n'est pas modifié au cours du délai *post-mortem.* Les 5 fragments ne résultent donc pas d'une activité enzymatiquc catabolique pouvant se produire après la mort.

### Analyse du tissu de patients à différents stades de la maladie d'Alzheimer.

### A) Diminution de la quantité de fragments APP-Cter au cours de l'Alzheimérisation

La figure 2B représente l'analyse, sur les mêmes échantillons et les mêmes immunoempreintes, de la pathologie tau selon la classification de Delacourte et al, 1999 (figure 2, B1) et des fragments APP-Cter (figure 2, B2) chez un sujet témoin (Pistes 1 et 2) et deux patients atteints de la maladie d'Alzheimer (pistes 3 à 6). Deux régions cérébrales ont été étudiées: l'hippocampe (Hip, pistes 1, 3 et 5) et le cortex occipital (Oc, pistes 2, 4 et 6). On note une diminution des fragments APP-Cter au cours de la maladie. Cette disparition est corrélée à la sévérité de l'atteinte neurodégénérative. En effet, on peut noter que les fragments APP-Cter disparaissent (figure 2, B2) dans les régions touchées par la pathologie tau, visualisée par la présence du triplet de protéines tau pathologiques de 69, 64 et 60 kDa (figure 2, B1) (Delacourte et al, (6)).

Un étude statistique, sur un grand nombre de patients, montre l'excellente corrélation entre l'avancée du processus pathologique, révélée par les stades de pathologie tau, et la diminution de la quantité de fragments APP-Cter dans les échantillons de tissu cérébral étudié.

Les quantités d'APP-Cter ont été déterminées dans le cortex de 4 groupes de patients: les cas témoins (Ctrl), les cas infracliniques (infraMA) qui correspondent à des patients non-déments, mais avec les lésions caractéristiques de la maladie d'Alzheimer, les cas cliniques de maladie d'Alzheimer (MA) et les cas familiaux autosomiques dominants de maladie d'Alzheimer (FAD.MA) (figure 2, C). Le test statistique non-parametrique de Mann-Whitney indique une diminution significative entre les groupes infraMA et MA par rapport aux témoins Ctrl (infraMA: p<0.03 ; MA p<0.002) . La diminution moyenne des APP-Cter est de 1.5 fois pour le groupe infraMA et 1.7 fois pour le groupe MA, par rapport au groupe témoin. La diminution des APP-Cer dans les groupes infraMA et MA n'est pas significativement différente. Ceci démontre que cette diminution de APP-Cter est un événement précoce de la physiopathologie Alzheimer.

L'étude statistique de la relation entre les niveaux d'expression des APP-Cter par rapport aux stades de la pathologie tau dans le cortex temporal des patients témoins et à différents stades de la maladie d'Alzheimer a été effectuée (figure 2, D). Une relation significative est observée entre les quantités d'APP-Cter détectées dans le cortex de chaque patient étudié et les différents stades de la pathologie tau, aussi bien dans le cortex temporal que dans le cortex occipital.

| | | | |
|---|---|---|---|
| Régression linéaire | | | |
| | n | r | p |
| cortex temporal | 26 | 0,614 | 0,0009 |
| | n | r | p |
| cortex occipital | 22 | 0,457 | 0,0324 |

### B) Transformation des fragments APP-Cter au cours du processus d'Alzheimérisation

Les fragments APP-Cter sont extraits préférentiellement dans deux phases: une solution tampon contenant un détergent non-ionique, le Triton X100 (Fraction F2), et une solution contenant un détergent ionique, le sodium dodecyl sulfate (SDS) (F3) (figure 3, A).

Il est observé des variations différentielles de solubilité des fragments APP-Cter au cours de l'Alzheimerization du tissu cérébral (figure 3B). On note une augmentation de la solubilité des fragments APP-Cter de masse moléculaire de 9,5 (fragment E) et 10,5 kDa (fragment D) dans la fraction F2 et une augmentation de l'insolubilité des fragments APP-Cter de masse moléculaire de 13,5 (fragment B) et 14,5 kDa (fragment A) dans la fraction F3.

### Analyse 2-D du tissu cérébral humain normal et pathologique

Les résultats sur 3 patients sont présentés sur la figure 4 (A à C)
- un patient témoin, sans lésions cérébrales de type Alzheimer (Stade 0 de Delacourte *et al,* (6)) (figure 4A)
- un patient au tout début de la phase clinique de maladie d'Alzheimer, au stade 6 de Delacourte et al (6) (figure 4B).

Les flèches indiquent les spots détectés par l'anticorps polyclonal APP-Cter-C17. Leurs masses moléculaires sont similaires à celles observées sur les immunoempreintes 1-D. Les points isoélectriques sont compris dans la gamme de pH de 4,5 à 7,5.

On peut noter une modification de la répartition des spots au cours du développement de la maladie d'Alzheimer (figure 4, C). Les patients à un stade plus avancé de la maladie d'Alzheimer n'ont pas pu être étudiés par cette approche, puisque les fragments ne sont plus détectables.

Les variants APP-Cter détectés par analyse 2D sont représentés schématiquement (figure 4, C) sur une grille calibrée en deux dimensions, l'axe des Y représentant les masses moléculaires et l'axe des X les points isoélectriques. L'ensemble a été étalonné avec des témoins internes de masse moléculaire et de point isoélectrique. Les témoins de points isoélectriques sont la gamma-énolase (47/4,94), l'alpha-actine (42/5,29) et l'alpha-énolase (47/6,99). Les points isoélectriques à 5,54 et 8,05 sont des standards internes du tissu cérébral.

Le schéma de la figure 4, représente la superposition des profils 2D des fragments APP-Cter détectés avec l'anticorps APP-Cter-C17 dans le tissu cérébral humain d'un sujet témoin et de patients Alzheimer au stade infraclinique de la maladie et au début du stade clinique de la maladie d'Alzheimer. Les spots, c'est à dire les différents variants isoélectriques de 2-D, observés à la fois dans le tissu sain et pathologique sont représentés par des cercles vides. Les spots spécifiques du tissu sain sont représentés par des cercles avec fond hachuré et ceux spécifiques du tissu Alzheimérisé par un fond noir.

Les valeurs des masses moléculaires et des points isoélectriques des spots modifiés au cours de la pathologie Alzheimer sont reportés dans le tableau 1.

### Analyses 1-D du tissu cérébral murin

Afin de mieux caractériser la spécificité du catabolisme et/ou du métabolisme de l'APP humain, les produits APP-Cter ont été analysés (de la même manière que dans le tissu cérébral humain) chez un rat et des souris non transgéniques mais également chez des souris transgéniques pour le gène de l'APP humain sauvage (APPwt) ou portant la mutation suédoise (APPsw). La figure 5A représente l'immunomarquage des fragments APP-Cter des souris témoins, et transgéniques, comparé au profil du tissu cérébral humain (figure 5A, piste 4).

Des dépôts identiques d'homogénats de tissu cérébral ont été déposés dans chaque puit d'électrophorèse. Les fragments APP-Cter ont été détectés avec l'anticorps polyclonal APP-Cter-C17. La piste 1 de la figure 5 correspond à l'analyse de la souris non transfectée (contrôle négatif), la piste 2 correspond à la souris avec une seule copie du gène humain APPwt, la piste 3 correspond à la souris transgénique avec le gène muté humain APPsw et la piste 4 correspond à du tissu cérébral humain témoin. Les flèches indiquent les masses moléculaires des bandes électrophorétiques détectées.

On peut noter que les souris témoins ou avec une seule copie du gène APPwt synthétisent essentiellement 4 fragments APP-Cter, tandis que les souris avec le gène APPsw et le tissu cérébral humain normal synthétisent 5 fragments APP-Cter, de masse moléculaire de 14,5 ; 13,5 ; 12 ; 10,5 et 9,5 kDa.

Ces résultats ont été vérifiés sur 5 souris non transgéniques, 2 souris transgéniques avec le gène APP humain de type sauvage et 5 souris transgéniques avec le gène APPsw.

On rapporte sur la figure 5B, piste 1 à 3, les résultats de l'immunoempreinte avec l'anticorps APP-Cter-C17 sur différentes lignées de souris transgéniques APPsw (fig 5B piste 1 à 3) parmi lesquelles on trouve (piste 3) la lignée APPsw de référence décrite par Hsiao et al., (8). Les souris transgéniques APPsw possèdent dont systématiquement un profil particulier, tel que décrit ici.

### Analyse 2-D des fragments APP-Cter du tissu cérébral de souris

On opère comme décrit pour le tissu cérébral humain (figure 4). Il est ainsi possible de caractériser les différents variants observés chez les souris Témoin (figure 6A), APPwt (figure 6B) et APPsw (figure 6C). On peut noter l'augmentation de l'expression des variants pour les souris APPsw et une modification du profil pour certains variants (figure 6). Les valeurs de masse moléculaire et de point isoélectrique sont reportées dans la figure 7A et le tableau 3.

### Analyse 1-D des modifications de l'APP humain dans des modèles cellulaires

### - modèles non neuronaux

Des cellules CHO (Chinese Hamster ovarian), transfectées de façon stable avec le gène APP humain de type sauvage (APPwt) ou le gène APPsw, ont été analysés selon le protocole décrit. Les résultats de la figure 8 montrent les fragments APP-Cter des cellules CHO, comparativement au tissu cérébral de souris trangéniques avec le gène APPwt ou APPsw.

Dans les cellules CHO APPwt (figure 8, piste 3), 6 bandes de masses moléculaires apparentes 16; 14,5; 13,5; 12; 10,5 et 9,5 kDa sont détectées. Les bandes majeures sont les bandes à 16; 14, 5; 13,5 et 9,5 kDa. Dans les cellules CHO APPsw (figure 8, piste 4), on observe une diminution du signal global surtout des fragments de 16 ; 13,5 et 9,5 kDa.

La comparaison avec les homogénats de tissu cérébral de souris transgénique APPwt (piste 1) et APPsw (piste 2) indique que les bandes détéctées dans les CHO sont communes à celles du tissu cérébral, excepté pour la bande à 16 kDa.

On notera que les souches de cellules gliales CCF, U118 et T98 possèdent un profil avec 6 bandes à 13,5, 12,10,5, 9,5, 9 et 8,5 kDa, avec une abondance des 3 bandes entre 9,5 et 8,5 kDa (figure 8B). Un profil similaire est observé dans souches cellulaires Hela, COS et SY-5Y transfectée avec l'APPsw (figure 8B).

En outre, la souche cellulaire épithéliale HT-29 présente un profil à 5 bandes à 14,5, 13,5, 12, 10,5, 9,5 et 9 kDa (figure 8: HT-29). La bande à 9 kDa qui est discrète dans les cellules épithéliales HT-29 devient plus abondante après traitement par le benzyl-O-GalNac qui inhibe la glycosylation et l'adressage des protéines glycosylées. Ceci démontre que la glycosylation est une modification post-traductionnelle qui régule l'expression et le profil des APP-Cter. Cette modification est donc un élément important à prendre en compte dans la recherche des transformations pathologiques moléculaires des APP-Cter au cours de l'Alzheimérisation.

### - Modèles neuronaux

Des cellules de neuroblastome humain (SKNSH-SY5Y) transfectées, de façon stable, avec les gène APPsw, APPwt, PS1 muté et Tau ont été analysées comparativement à des cellules non transfectées. Les résultats sont représentés sur la figure 9. Dans les cellules non transfectées, le signal APP-Cter correspond à la bande de 10,5 kDa (figure 9 : Native). En revanche, 6 fragments à 13,5, 12, 10,5, 9,5, 9 et 8,5 kDa sont parfaitement identifiables dans les cellules SKNSH non-différenciées, transfectées avec APPsw (figure 9 : APPsw). On retrouve également le profil des 6 bandes avec les cellules transfectées avec APP wt (figure 9: wild type). La transfection des cellules SY5Y avec le gène PS1 muté provoque une augmentation des 5 bandes, de la bande à 9,5 kDa qui est ici abondante, et également du fragment de 6,5 kDa, libéré par l'activité gamma secrétase.

D'autres lignées neuronales natives possèdent également des profils du type SKNSH natif, comme les cellules Kelly, ou, par ailleurs, comme les cellules SKNSH transfectées avec un gène muté de APP ou PS1, comme les cellules NT2 et hNT.

La différentiation pendant 14 jours des cellules SY5Y provoque un changement de profil, avec l'apparition de la bande gamma à 6,5 kDa et d'une diminution de la bande à 10,5 kDa. Dans les mêmes conditions, les cellules transfectées avec le gène tau 3R et non différenciées voient l'apparition de la bande à 13,5 kDa, correspondant à une augmentation de la libération des fragments "béta secrétase". Dans ces cellules différenciées, on observe la disparition de la bande gamma à 6,5 kDa.

Ceci démontre que des gènes sont capables de moduler l'expression et la répartition relative des fragments APP-Cter. Ces modèles ont donc la potentialité de modéliser les modifications pathologiques des fragments APP-Cter, tels qu'observé dans le tissu cérébral humain.

### Analyse des fragments APP-Cter des globules blancs humains

Des culots de globules blancs humains ont été préparés selon un protocole standard utilisé en laboratoire d'analyse (médical ou hospitalier). Ces culots ont été traités comme décrit et analysés par immunoempreintes avec l'anticorps APP-Cter-C17. Les résultats sont rapportés sur la figure 10A où les pistes 1 à 5 correspondent aux témoins et les pistes 6 à 11 à des échantillons provenant de patients atteints de la maladie d'Alzheimer. Le sexe et l'âge sont mentionnés (figure 10A : sexe.âge).

L'anticorps APP-Cter-C17 détecte trois fragments de masse moléculaire de 14 kDa, 10,5 kDa et 9,5 kDa.

Une analyse 1-D comparative entre globules blancs de sujets témoins et de patients diagnostiqués maladie d'Alzheimer a permis de mettre en évidence (comme dans l'étude du tissu cérébral humain) une diminution des fragments APP-Cter dans les globules blancs des malades (pistes 6 à 11) par rapport aux globules blancs de témoins (pistes 1 à 5).

Les variants des fragments APP-Cter des globules blancs ont également été analysés en 2-D (figure 10B). Les valeurs de masse moléculaire et de point isoélectrique de chaque spot détecté ont été rapportées sur la figure 7C et le tableau 2.

### Caractérisation des fragments APP-Cter avec différentes sondes immunologiques

Un anticorps dirigé contre l'extrémité N-terminale du peptide Aβ permet de localiser le fragment de coupure de la béta-secrétase. Il s'agit de l'anticorps FCA18 (4). Le fragment 14 kDa des globules blancs est détecté par cet anticorps, indiquant qu'il s'agit du fragment APP-Cter produit par la béta secrétase (figure 11B). Dans le tissu cérébral, les deux bandes A (14,5 kDa) et B (13,5 kDa) sont reconnues par WO2, un anticorps monoclonal (Abeta, GmBH, Heidelberg, Germany) dirigé contre la partie 5-11 du peptide Aβ tandis que la bande 3 est détectée par FCA18, ce qui indique que les bandes A et B sont générées par une coupure située au niveau de la région beta secrétase (voir figure 1). Par ailleurs, l'interrogation des bases de données (SwissProt par exemple) indique que le fragment APP-Cter de masse moléculaire de 9,5 kDa (figure 11) et de point isoélectrique 6,99 correspond au fragment alpha secrétase, comme représenté dans la figure 1. Les deux fragments APP-Cter majeurs des globules blancs qui sont modifiés au cours de la maladie d'Alzheimer ont donc été identifiés (figure 11, A et B).

Par ailleurs, certains sites communs aux 5 fragments A à C peuvent être modifiés différentiellement. Ainsi, par exemple, un anticorps polyclonal reconnaissant spécifiquement la thréonine 668 phosphorylée reconnaît préférentiellement les bandes A, C et D. La méthode décrite ici permet de caractériser les modifications post-traductionnelles liées au processus dégénératif. Elle débouche de ce fait sur la possibilité d'élaborer de nouveaux outils immunologiques qui viendront compléter avantageusement le kit de diagnostic de la transformation pathologique des fragments APP-Cter.

### La phosphorylation des fragments APP-Cter est un élément déterminant de leur transformation pathologique

Il se produit une modification du métabolisme et de la phosphorylation des APP-Cter dans la maladie d'Alzheimer. Les APP-Cter du tissu cérébral d'un sujet témoin et d'un patient Alzheimer ont été immunoprécipités à l'aide de l'anticorps APP-CterC17 et les 5 fragments à 14,5, 13,5, 12, 10,5, et 9,5 kDa (fragments A, B, C, D et E) sont révélés après électrophorèse en gel Tris-Tricine avec le même anticorps (figure 13, pistes 1 et 2). Les fragments APP-Cter immunoprécipités ont été déphosphorylés à l'aide de la phosphatase alkaline d'intestin de veau (figure 13, pistes 3 et 4) puis révélés avec l'anticorps APP-CterC17 suivant le même protocole. Trois fragments majeurs sont détectés dans l'homogénat témoin à 13,5 ; 10,5 et 9,5 kDa. Ces trois fragments sont également détectés dans l'homogénat Alzheimer. Cependant le fragment à 13,5 kDa est en plus faible quantité alors que les fragments à 10,5 et 9,5 kDa sont en plus grande quantité. Ce résultat montre l'accumulation des fragments générés par la coupure alpha-secrétase, mais également leur phosphorylation plus importante.

Les modifications de l'indice de phosphorylation sont démontrées en particulier avec l'anticorps anti-thréonine 668 phosphorylée (figure 13, pistes 5 et 6). Les fragments APP-Cter phosphorylés sur la Thréonine 668 ont été immunoprécipités et révélés à l'aide de l'anticorps APP-CterC17 après électrophorèse en gel Tris-Tricine. Les fragments à 14,5 ; 12 et 10,5 sont détectés dans l'homogénat témoin. Dans l'homogénat Alzheimer ce sont essentiellement les fragments à 12,5 et 10,5 kDa qui sont détectés en grande quantité. Ce résultat confirme que les fragments APP-Cter C et D sont plus phosphorylés et en plus grande quantité dans la phase précoce de la maladie d'Alzheimer, et que parallèlement le fragment A est peu ou pas phosphorylé au site 668.

### Récapitulatif

Des variants particulièrement informatifs pour la mise en oeuvre de l'invention sont rapportés dans les tableaux récapitulatifs (figures 14 à 16). Ces différents variants constituent des marqueurs de grand intérêt pour détecter les modifications pathologiques de l'APP, aussi bien dans les tissus neuronaux que dans les tissus non-neuronaux, et en particulier les lymphocytes.

La phosphorylation sur les thréonines, sérines et tyrosines des fragments APP-Cter (figure 1) contribuent au profil général des APP-Cter. En premier lieu, on observe une différence de l'indice de phosphorylation de ces différents fragments APP-Cter, comme le montre en particulier la thréonine 668 phosphorylée (figures 11 et 13). Ensuite, le profil des fragments APP-Cter déphosphorylés est modifié, avec notamment la disparition de la bande de 14,5 kDa, et le nouveau profil ressemble alors à celui des modèles cellulaires SKNSH transfectés avec APPsw (Figure 13).

La déphosporylation des fragments APP-Cter révèle par ailleurs un changement de profil entre le tissu normal et le tissu Alzheimer, avec notamment une accumulation des bandes C,D,E des fragments APP-Cter, correspondant aux produits de coupure de l'alpha secrétase (figure 13). La phosphorylation est donc un événement post-traductionnel important, et un marqueur de la transformation pathologique des fragments APP-Cter.

Cette modification se reflète également sur le fragment gamma-secrétase, de 6,5 kDa, qui est modifié dans son expression par les gènes impliqués dans la maladie d'Alzheimer, comme le montrent les modèles cellulaires (figure 9).

La détection des fragments APP-Cter permet la définition d'un indice de transformation pathologique, applicable au tissu humain neuronal (figure 12A: tissu cérébral humain) ou non-neuronal (figure 12B: lymphocytes humains), ainsi qu'aux modèles expérimentaux, animaux (figure 12C: tissu cérébral de souris transgénique APPsw et wt) ou cellulaires, neuronaux (figure 12D: cellules de neuroblastome) ou non-neuronaux (cellules CHO, COS,...). Cet indice varie de 0 (tissu sain) à 100% (tissu pathologique Alzheimérisé) (figure 12).

### RÉFÉRENCES

1. De Strooper B, Konig G (1999) Alzheimer's disease. A firm base for drug development [news] [comment]. Nature, 402, 471-2.
2. Sigurdsson EM, Permanne B, Soto C, Wisniewski T, Frangione B (2000) In vivo reversal of amyloid-beta lesions in rat brain. J Neuropathol Exp Neurol, 59, 11-7.
3. Schenk D, Barbour R, Dunn W, Gordon G, Grajeda H, Guido T, Hu K, Huang J, Johnson-Wood K, Khan K, Kholodenko D, Lee M, Liao Z, Lieberburg I, Motter R, Mutter L, Soriano F, Shopp G, Vasquez N, Vandevert C, Walker S, Wogulis M, Yednock T, Games D, Seubert P. (1999) Immunization with amyloid-beta attenuates Alzheimer-disease-like pathology in the PDAPP mouse. Nature 400:173-7.
4. Ancolio K, Dumanchin C, Barelli H, Warter JM, Brice A, Campion D, et al. (1999) Unusual phenotypic alteration of beta amyloid precursor protein (betaAPP) maturation by a new Val-715 --> Met betaAPP-770 mutation responsible for probable early-onset Alzheimer's disease. Proc Natl Acad Sci U S A, 96, 4119-24.
5. Vaitukaitis J, Robbins JB, Nieschlag E, Ross GT (1971) A method for producing specific antisera with small doses of immunogen. J Clin Endocrinol Metab, 33, 988-91.
6. Delacourte A, David JP, Sergeant N, Buee L, Wattez A, Vermersch P, et al. (1999) The biochemical pathway of neurofibrillary degeneration in aging and Alzheimer's disease, Neurology, 52, 1158-65.
7. Duff K, Eckman C, Zehr C, Yu X, Prada CM, Perez-tur J, et al. (1996) Increased amyloid-beta42 (43) in brains of mice expressing mutant presenilin 1. Nature, 383, 710-3.
8. Hsiao K, Chapman P, Nilsen S, Eckman C, Harigaya Y, Younkin S, et al. (1996) Correlative memory deficits, Abeta elevation, and amyloid plaques in transgenic mice. Science, 274, 99-102.
9. Laemmli UK (1970) Cleavage of structural proteins during the assembly of the head of bacteriophage T4. Nature, 227, 680-5.
10. Schagger H, von Jagow G (1987) Tricine-sodium dodecyl sulfate-polyacrylamide gel electrophoresis for the separation of proteins in the range from 1 to 100 kDa. Anal Biochem, 166, 368-79.

## Revendications

1. Méthode pour détecter, dans un échantillon à analyser, des modifications pathologiques de la protéine APP endogène, **caractérisée en ce qu'**on détermine une diminution de la quantité de marqueurs, et/ou une différence de solubilité des marqueurs, lesdits marqueurs étant constitués par des fragments cataboliques et/ou métaboliques de la partie carboxy-terminale de l'APP, fragments APP-Cter, spécifiquement modifiés dans les situations neurodégénératives où l'APP participe à l'étiologie.

2. Méthode selon la revendication 1, **caractérisée en ce qu'**elle comprend l'utilisation, comme marqueurs de dysfonctionnement de l'APP, de 1 à 7 fragments choisis parmi ceux de 14,5 ; 13,5; 12 ; 10,5; 9,5 ; 9 et 6,5 kDa et des isovariants d'isoélectrofocalisation constituant ces fragments.

3. Méthode selon la revendication 1, **caractérisée en ce qu'**elle comprend l'utilisation, comme marqueurs de dysfonctionnement de l'APP, de 1 à 8 fragments choisis parmi ceux de 14, 5 ; 13, 5 ; 12 ; 10,5 ; 9, 5 ; 9 ; 8,5 et 6,5 kDa et des isovariants d'isoélectrofocalisation constituant ces fragments.

4. Méthode selon l'une quelconque des revendications 1 à 3, **caractérisée en ce qu'**on utilise une diminution de la quantité de marqueurs.

5. Méthode selon la revendication 4, **caractérisée en ce qu'**elle comprend l'utilisation, comme marqueurs de dysfonctionnement de l'APP, de 5 fragments de 14,5 ; 13,5 ; 12 ; 10,5 et 9,5 kDa et des isovariants d'isoélectrofocalisation constituant ces fragments, en particulier ceux qui sont modifiés au cours des situations pathologiques chez l'homme ou dans les modèles expérimentaux.

6. Méthode selon la revendication 5, **caractérisée en ce qu'**elle comprend l'utilisation additionnelle d'un fragment de 6,5 kDa et des isovariants d'isoélectrofocalisation constituant ce fragment, en particulier ceux qui sont modifiés au cours des situations pathologiques chez l'homme ou dans les modèles expérimentaux.

7. Méthode selon la revendication 5, **caractérisée en ce qu'**elle comprend l'utilisation additionnelle d'un fragment de 9 kDa et des isovariants d'isoélectrofocalisation constituant ce fragment, en particulier ceux qui sont modifiés au cours des situations pathologiques chez l'homme ou dans les modèles expérimentaux.

8. Méthode selon la revendication 4, **caractérisée en ce qu'**elle comprend l'utilisation, comme marqueurs de dysfonctionnement de l'APP, de 8 fragments de 14,5 ; 13,5 ; 12 ; 10,5 ; 9,5 ; 9 ; 8,5 et 6,5 kDa et des isovariants d'isoélectrofocalisation constituant ces fragments, en particulier ceux qui sont modifiés au cours des situations pathologiques chez l'homme ou dans les modèles expérimentaux.

9. Méthode selon l'une quelconque des revendications 1 à 3, **caractérisée en ce qu'**on utilise une différence de solubilité.

10. Méthode selon la revendication 9, **caractérisée en ce que** la solubilité des fragments de 14,5 et/ou de 13,5 est diminuée.

11. Méthode selon la revendication 9, **caractérisée en ce que** la solubilité des fragments de 10,5 et/ou de 13,5 est augmentée.

12. Méthode pour détecter, dans un échantillon à analyser, des modifications pathologiques de la protéine APP endogène, **caractérisée en ce qu'**on utilise une différence de charge ou de point isoélectrique des marqueurs, lesdits marqueurs étant constitués par des fragments cataboliques et/ou métaboliques de la partie carboxy-terminale de l'APP, fragments APP-Cter, spécifiquement modifiés dans les situations neurodégénératives où l'APP participe à l'étiologie, la différence de charge ou de point isoélectrique correspondant à une diminution de la phosphorylation des fragments APP-Cter β (bandes A, B) et optionnellement, une augmentation de la phosphorylation des fragments APP-Cter α (bandes C, D, E).

13. Méthode selon l'une quelconque des revendications 1 à 12, **caractérisée en ce que** les échantillons à analyser comprennent des tissus ou des cellules neuronales, ou des tissus ou des cellules non neuronales, par exemple des liquides biologiques comme le liquide céphalo-rachicien, le sang et tout ou partie de ses éléments figurés tels que les leucocytes.

14. Méthode selon l'une quelconque des revendications 1 à 12, **caractérisée en ce qu'**on utilise un anticorps polyclonal et/ou monoclonal dirigé contre la partie carboxy-terminale de l'APP.

15. Méthode selon la revendication 14, **caractérisée en qu'**on utilise en complément un jeu d'anticorps polyclonaux et/ou monoclonaux révélant les modifications post-traductionnelles de la transformation pathologique des fragments APP-Cter, en particulier une modification de type phosphorylation et/ou méthylation et/ou acétylation et/ou glycosylation.

16. Méthode selon la revendication 15, **caractérisée en que** le jeu d'anticorps polyclonaux et/ou monoclonaux utilisé permet de révéler les phosphorylations et la variation d'expression des fragments de 14,5 ; 12 et 10,5 kDa.

17. Méthode selon l'une quelconque des revendications 1 à 16, **caractérisée en ce qu'**on évalue le degré de la pathologie en affectant un indice aux fragments identifiés dans l'échantillon étudié, cet indice étant attribué par rapport à un référentiel où l'indice 0 (zéro) correspond à la détection dans le tissu sain et l'indice 100 (cent), dans le tissu pathologique.

18. Méthode selon l'une quelconque des revendications 1 à 17, **caractérisée en ce qu'**elle est utilisée pour le diagnostic et le suivi d'un processus neurodégénératif, la mise au point de modèles animaux et le criblage de médicaments efficace contre les pathologies de l'APP.

19. Méthode selon la revendication 18, **caractérisée en ce qu'**elle est utilisée pour la détection du processus d'Alzheimer ou pour le diagnostic différentiel de pathologies neurodégénératives voisines de la maladie d'Alzheimer, comme la démence à corps de Lewy ou les angiopathies amyloïdes.

20. Application de la méthode selon l'une quelconque des revendications 1 à 19, pour valider des modèles expérimentaux, cellules ou modèle animal, de la maladie d'Alzheimer.

21. Application de la méthode selon l'une quelconque des revendications 1 à 19, au diagnostic de pathologies dégénératives de type Alzheimer sur le tissu humain et sur les modèles expérimentaux.

22. Application de la méthode selon l'une quelconque des revendications 1 à 19, pour suivre l'effet et/ou pour corriger un traitement thérapeutique basé sur le catabolisme et le métabolisme de l'APP.

## Claims

1. A method for detecting, in a sample to be analyzed, pathological modifications of the endogenous APP protein, comprising determining a reduction in the quantity of markers and/or a difference of solubility of the markers, said markers being constituted by catabolic and/or metabolic fragments of the carboxy-terminal part of the APP, APP-Cter fragments, specifically modified in neurodegenerative situations where the APP participates in the etiology.

2. The method according to claim 1, comprising the use, as APP dysfunction markers, from 1 to 7 fragments chosen from those from 14.5; 13.5; 12; 10.5; 9.5; 9 and 6.5 kDa and isovariants of isoelectric focusing constituting these fragments.

3. The method according to claim 1, comprising using, as APP dysfunction markers, from 1 to 8 fragments chosen from those of 14.5; 13.5; 12; 10.5; 9.5; 9; 8.5 and 6.5 kDa and isovariants of isoelectric focusing constituting these fragments.

4. The method according to anyone of claims 1 to 3, comprising using a reduction in the quantity of markers.

5. The method according to claim 4, comprising the use, as APP dysfunction markers, of 5 fragments of 14.5; 13.5; 12; 10.5 and 9.5 kDa and isovariants of isoelectric focusing constituting these fragments, in particular those which are modified during pathological situations in humans or in experimental models.

6. The method according to claim 5, comprising the additional use of a fragment of 6.5 kDa and isovariants of isoelectric focusing constituting this fragment, in particular those which are modified during pathological situations in humans or in experimental models.

7. The method according to claim 5, comprising the additional use of a fragment of 9 kDa and isovariants of isoelectric focusing constituting this fragment, in particular those which are modified during pathological situations in humans or in experimental models.

8. The method according to claim 4, comprising the use, as APP dysfunction markers, of 8 fragments of 14.5; 13.5; 12; 10.5; 9.5; 9; 8.5 and 6.5 kDa and isovariants of isoelectric focusing constituting these fragments, in particular those which are modified during pathological situations in humans or in experimental models.

9. The method according to anyone of claims 1 to 3, comprising using a difference of solubility.

10. The method according to claim 9, wherein the solubility of fragments of 14.5 and/or 13.5 is reduced.

11. The method according to claim 9, wherein the solubility of fragments of 10.5 and/or 9.5 is increased.

12. A method for detecting in a sample to be analyzed, pathological modifications of the endogeneous APP protein, comprising using a difference of charge or isoelectric point of the markers, said markers being constituted by catabolic and/or metabolic fragments of the carboxy-terminal part of the APP, APP-Cter fragments, specifically modified in neurodegenerative situations where the APP participates in the etiology, the difference of charge or isoelectric point corresponding to the diminution of the phosphorylation of APP-Cter β fragments (bands A, B) and optionally, an increase of the phosphorylation of APP-Cter α fragments (bands C, D, E).

13. The method according to anyone of claims 1 to 12, wherein the samples to be analyzed comprise neuronal tissues or cells, or non-neuronal tissues or cells, for example biological liquids such as cerebrospinal fluid, blood, and all or part of its formed elements such as leukocytes.

14. The method according to anyone of claims 1 to 12, comprising using a polyclonal and/or monoclonal antibody to the carboxy-terminal region of the APP.

15. The method according to claim 14, comprising additionally using a set of polyclonal and/or monoclonal antibodies, revealing the post-translational modifications of the pathological transformation of the APP-Cter fragments, in particular a phosphorylation and/or methylation and/or acetylation and/or glycosylation-type modification.

16. The method according to claim 15, wherein the set of polyclonal and/or monoclonal antibodies used, makes it possible to reveal the phosphorylations and variation of expression of the 14.5; 12 and 10.5 kDa fragments.

17. The method according to anyone of claims 1 to 16, comprising evaluating the degree of the pathology by allocating an index to the fragments identified in the sample studied, this index being assigned with respect to a system of reference where the index 0 (zero) corresponds to detection in healthy tissue, and the index 100 (one hundred) in pathological tissue.

18. The method according to anyone of claims 1 to 17, comprising using for diagnosis and monitoring of neurodegenerative diseases, the development of animal models and the screening of medicaments effective against the APP pathologies.

19. The method according to claim 18, **characterized in that** it is used for detection of the Alzheimer's process or for the differential diagnosis of neurodegenerative pathologies related to Alzheimer's disease, such as dementia with Lewy bodies or the amyloid angiopathies.

20. Use of the method according to anyone of claims 1 to 19, in order to validate experimental, cells or animal models, of Alzheimer's disease.

21. Use of the method according to anyone of claims 1 to 19, for the diagnosis of Alzheimer-type neurodegenerative pathologies in human tissue and experimental models.

22. Use of the method according to anyone of claims 1 to 19, for monitoring the effect of and/or for correcting a therapeutic treatment based on the catabolism and metabolism of APP.

## Patentansprüche

1. Verfahren zum Nachweisen von pathologischen Modifikationen des endogenen Proteins APP in einer zu analysierenden Probe, **dadurch gekennzeichnet, dass** man eine Verringerung der Menge von Markern und/oder einen Unterschied in der Löslichkeit der Marker verwendet, wobei die Marker aus katabolischen und/oder metabolischen Fragmenten des carboxyterminalen Teils von APP bestehen, APP-Cter-Fragmenten, die in neurodegenerativen Situationen, bei denen APP an der Ätiologie beteiligt ist, spezifisch modifiziert sind.

2. Verfahren gemäß Anspruch 1, **dadurch gekennzeichnet, dass** es die Verwendung von 1 bis 7 Fragmenten, die aus solchen von 14,5; 13,5; 12; 10,5; 9,5; 9 und 6,5 kDa und von Isovarianten der isoelektrischen Fokussierung, aus denen diese Fragmente bestehen, ausgewählt sind, als Marker der Dysfunktion von APP umfasst.

3. Verfahren gemäß Anspruch 1, **dadurch gekennzeichnet, dass** es die Verwendung von 1 bis 8 Fragmenten, die aus solchen von 14,5; 13,5; 12; 10,5; 9,5; 9; 8,5 und 6,5 kDa und von Isovarianten der isoelektrischen Fokussierung, aus denen diese Fragmente bestehen, ausgewählt sind, als Marker der Dysfunktion von APP umfasst.

4. Verfahren gemäß einem der Ansprüche 1 bis 3, **dadurch gekennzeichnet, dass** man eine Verringerung der Menge von Markern verwendet.

5. Verfahren gemäß Anspruch 4, **dadurch gekennzeichnet, dass** es die Verwendung von 5 Fragmenten von 14,5; 13,5; 12; 10,5 und 9,5 kDa und von Isovarianten der isoelektrischen Fokussierung, aus denen diese Fragmente bestehen, insbesondere solchen, die im Laufe von pathologischen Situationen beim Menschen oder in experimentellen Modellen modifiziert werden, als Marker der Dysfunktion von APP umfasst.

6. Verfahren gemäß Anspruch 5, **dadurch gekennzeichnet, dass** es die zusätzliche Verwendung eines Fragments von 6,5 kDa und von Isovarianten der isoelektrischen Fokussierung, aus denen diese Fragmente bestehen, insbesondere solchen, die im Laufe von pathologischen Situationen beim Menschen oder in experimentellen Modellen modifiziert werden, umfasst.

7. Verfahren gemäß Anspruch 5, **dadurch gekennzeichnet, dass** es die zusätzliche Verwendung eines Fragments von 9 kDa und von Isovarianten der isoelektrischen Fokussierung, aus denen diese Fragmente bestehen, insbesondere solchen, die im Laufe von pathologischen Situationen beim Menschen oder in experimentellen Modellen modifiziert werden, umfasst.

8. Verfahren gemäß Anspruch 4, **dadurch gekennzeichnet, dass** es die Verwendung von 8 Fragmenten von 14,5; 13,5; 12; 10,5; 9,5; 9; 8,5 und 6,5 kDa und von Isovarianten der isoelektrischen Fokussierung, aus denen diese Fragmente bestehen, insbesondere solchen, die im Laufe von pathologischen Situationen beim Menschen oder in experimentellen Modellen modifiziert werden, als Marker der Dysfunktion von APP umfasst.

9. Verfahren gemäß einem der Ansprüche 1 bis 3, **dadurch gekennzeichnet, dass** man einen Unterschied in der Löslichkeit verwendet.

10. Verfahren gemäß Anspruch 9, **dadurch gekennzeichnet, dass** die Löslichkeit der Fragmente von 14,5 und/oder 13,5 verringert ist.

11. Verfahren gemäß Anspruch 9, **dadurch gekennzeichnet, dass** die Löslichkeit der Fragmente von 10,5 und/oder 13,5 erhöht ist.

12. Verfahren zum Nachweisen von pathologischen Modifikationen des endogenen Proteins APP in einer zu analysierenden Probe, **dadurch gekennzeichnet, dass** man einen Unterschied in der Ladung oder im isoelektrischen Punkt der Marker verwendet, wobei die Marker aus katabolischen und/oder metabolischen Fragmenten des carboxyterminalen Teils von APP bestehen, APP-Cter-Fragmenten, die in neurodegenerativen Situationen, bei denen APP an der Ätiologie beteiligt ist, spezifisch modifiziert sind, wobei der Unterschied in der Ladung oder im isoelektrischen Punkt einer Verringerung der Phosphorylierung der β-APP-Cter-Fragmente (Banden A, B) und gegebenenfalls einer Erhöhung der Phosphorylierung der α-APP-Cter-Fragmente (Banden C, D, E) entspricht.

13. Verfahren gemäß einem der Ansprüche 1 bis 12, **dadurch gekennzeichnet, dass** die zu analysierenden Proben neuronale Gewebe oder Zellen oder nichtneuronale Gewebe oder Zellen umfassen, zum Beispiel biologische Flüssigkeiten, wie Liquor, Blut und alle oder einen Teil seiner Blutkörperchen, wie Leukocyten.

14. Verfahren gemäß einem der Ansprüche 1 bis 12, **dadurch gekennzeichnet, dass** man einen polyklonalen und/oder monoklonalen Antikörper verwendet, der gegen den carboxyterminalen Teil von APP gerichtet ist.

15. Verfahren gemäß Anspruch 14, **dadurch gekennzeichnet, dass** man ergänzend eine Menge von polyklonalen und/oder monoklonalen Antikörpern verwendet, die die posttranslationalen Modifikationen der pathologischen Transformation der APP-Cter- Fragmente nachweisen, insbesondere eine Modifikation des Typs Phosphorylierung und/oder Methylierung und/oder Acetylierung und/oder Glycosylierung.

16. Verfahren gemäß Anspruch 15, **dadurch gekennzeichnet, dass** die Menge der verwendeten polyklonalen und/oder monoklonalen Antikörper es erlaubt, die Phosphorylierungen und die Variation der Expression der Fragmente von 14,5; 12 und 10,5 kDa nachzuweisen.

17. Verfahren gemäß einem der Ansprüche 1 bis 16, **dadurch gekennzeichnet, dass** man den Grad der Pathologie bewertet, indem man einen Index für die in der untersuchten Probe identifizierten Fragmente vorsieht, wobei dieser Index in Bezug auf einen Referenzwert zugeordnet wird, bei dem der Index 0 (null) dem Nachweis in gesundem Gewebe entspricht und der Index 100 (hundert) dem Nachweis in pathologischem Gewebe entspricht.

18. Verfahren gemäß einem der Ansprüche 1 bis 17, **dadurch gekennzeichnet, dass** es für die Diagnose und Überwachung eines neurodegenerativen Vorgangs, die Entwicklung von Tiermodellen und die Suche nach wirksamen Medikamenten (Screening) gegen die Pathologien des APP verwendet wird.

19. Verfahren gemäß Anspruch 18, **dadurch gekennzeichnet, dass** es für den Nachweis des Alzheimer-Vorgangs oder für die Differentialdiagnose von neurodegenerativen Pathologien, die der Alzheimer-Krankheit ähnlich sind, wie Lewy-Body-Demenz oder Amyloid-Angiopathien, verwendet wird.

20. Anwendung des Verfahrens gemäß einem der Ansprüche 1 bis 19 für die Bewertung von experimentellen Modellen, Zellen oder Tiermodellen der Alzheimer-Krankheit.

21. Anwendung des Verfahrens gemäß einem der Ansprüche 1 bis 19 auf die Diagnose von degenerativen Pathologien des Alzheimer-Typs an menschlichem Gewebe und an experimentellen Modellen.

22. Anwendung des Verfahrens gemäß einem der Ansprüche 1 bis 19 zur Überwachung der Wirkung und/oder zur Korrektur einer therapeutischen Behandlung auf der Basis des Katabolismus und Metabolismus von APP.
